# EUROPEAN PATENT SPECIFICATION

(11) **EP 1 996 508 B1**
(45) Date of publication and mention of the grant of the patent: **13.07.2016**
(21) Application number: 07715412.8
(22) Date of filing: 27.02.2007
(51) Int. Cl.: B82B 3/00, A61K 41/00, A61K 47/48, A61L 2/238, A23L 3/358, A61F 2/14, A61L 27/04

(54) **Preparation method for water-soluble magnetic or metal oxide nanoparticles coated with ligands, and usage thereof**
Herstellungsverfahren für wasserlösliche, mit Liganden beschichtete Magnet- oder Metalloxidnanoteilchen, und Verwendung davon
Procédé de préparation pour des nanoparticules magnétiques ou d'oxyde métallique hydrosolubles couvertes de ligands, et l'utilisation desdites nanoparticules

(30) Priority: 27.02.2006 KR 20060018921
(43) Date of publication of application: 03.12.2008
(73) Proprietor: Industry-Academic Cooperation Foundation, Yonsei University, Seodaemun-gu Seoul 120-749 (KR)
(72) Inventor: CHEON, Jin Woo, Seoul, 158-724 (KR); JUN, Young Wook, Gyeonggi-do, 411-570 (KR); CHOI, Jin Sil, Seoul, 120-823 (KR)
(74) Representative: Eisenführ Speiser
(86) International application number: PCT/KR2007/001001
(87) International publication number: WO 2007/097605

(56) References cited:
- WO-A1-2004/096190
- WO-A1-2006/025627
- WO-A2-01/19405
- WO-A2-02/41826
- WO-A2-2004/020453
- KR-A- 20030 096 097
- US-A1- 2002 120 165
- US-A1- 2004 058 457
- US-A1- 2004 077 844
- US-A1- 2005 165 120
- US-B1- 6 767 635

## Description

### Technical Field

The present invention relates to water-soluble water-soluble metal oxide nanoparticles, and a preparation method and usage thereof, and more particularly, to water-soluble water-soluble metal oxide nanoparticles, in which the metal oxide nanoparticles are coated with phase transfer ligands composed of an adhesive region, an adhesive region-crosslinking region, or an adhesive region-reactive region, to a method of preparing water-soluble metal oxide nanoparticles from water-insoluble metal oxide nanoparticles through phase transfer, and to the usage of the water-soluble metal oxide nanoparticles in various fields, including the separation and detection of materials, the diagnosis and treatment of diseases, and the decomposition of microorganisms and contaminants.

### Background Art

Generally, magnetic nanoparticles and metal oxide nanoparticles, which exhibit intrinsic magnetic, optical and electrical properties, are expected to function as important constituents in next-generation electronic, information, and communication technology and biotechnology. In particular, such magnetic or metal oxide nanoparticles are used in various biotechnology fields, including imaging probes such as magnetic resonance imaging, biosensors including giant magnetoresistive sensors, micro-hydraulic system sensors, the delivery of drugs/genes, and magnetic thermotherapy. They are expected to stimulate innovative development in ultrahigh-speed, high-resolution, and high-sensitivity diagnostic methods and pinpointing therapy.

Specifically, magnetic nanoparticles may be used as a diagnostic probe in molecular magnetic resonance imaging. The magnetic nanoparticles shorten the spin-spin relaxation time of the hydrogen atoms in water molecules around the nanoparticles, thus amplifying the magnetic resonance imaging signal, and therefore have been widely used in molecular imaging diagnosis to date.

Further, the magnetic nanoparticles act as the probe material of a giant magnetoresistor (GMR). When the magnetic nanoparticles sense biomolecules, which are patterned on the surface of the giant magnetoresistive biosensor, in order to be bound thereto, the current signal of the giant magnetoresistive sensor is changed by the magnetic nanoparticles, therefore selectively detecting the biomolecules (US 6,452,763; US 6,940,277; US 6,944,939; US 2003/0133232).

Further, magnetic nanoparticles may be applied to the separation of biomolecules. For example, in the case where cells for exhibiting specific biomarkers are mixed with other cells, when the magnetic nanoparticles that are to be selectively bound to the specific biomarkers are added and an external magnetic field is applied thereto, only desired cells are separated in the direction of the magnetic field (US 4,554,088; US 5,665,582; US 5,508,164; US 2005/0215687). Furthermore, these particles may be applied to the separation of various biomolecules, including proteins, antigens, peptides, DNA, RNA, and viruses.

In addition, magnetic nanoparticles are applied to magnetic micro-hydraulic sensors in order to separate and detect the biomolecules. Very small channels are made on chips, and the magnetic nanoparticles are made to flow in the channels, thereby detecting and separating the biomolecules in the hydraulic system on a micro scale.

In addition, magnetic nanoparticles may be used in biomedical applications through the delivery of drugs or genes. The drugs or genes are attached to the magnetic nanoparticles through chemical bonding or adsorption, and are then moved to a desired area using an external magnetic field. This makes it possible to release the drugs and genes at the desired portion thus exhibiting selective therapeutic effects (US 6,855,749).

Another biomedical application using magnetic nanoparticles is thermotherapy using magnetic spin energy (US 6,530,944; US 5,411,730). When the alternating current of external radio frequency radiation is applied to the magnetic nanoparticles, heat is generated through spin flipping of magnetic nanoparticles. As such, since the temperature around the nanoparticles is 40°C or higher, cells die due to excess heat, and ultimately cells, such as cancer cells, may be selectively killed.

The magnetic nanoparticles are implanted in parts of the bodies to thus substitute for bio-organs. For example, iron oxide nanoparticles are mixed with a shape memory polymer, an external magnetic field is applied to create heat, and the form of the shape memory polymer is changed by the heat, thereby making it possible to develop an artificial retina (Proceedings of National Academy of Science USA, 2006, Vol.103, p.3540).

Metal oxide nanoparticles are also used in various fields. For example, since titanium oxide nanoparticles, having intrinsic photoelectron catalytic activity, form photoelectrons using external light and thus exhibit disinfecting activity, they may be used not only for various fresh goods to be disinfected but also for killing cancer cells and pathogens.

To apply the magnetic nanoparticles or metal oxide nanoparticles to the end uses mentioned above, 1) the nanoparticles should have excellent physical/chemical properties. Nanoparticles should be also 2) stablely transported and dispersed *in vivo,* that is, in a water-soluble environment, and 3) be easily conjugated to bioactive materials. Accordingly, in order to satisfy the above conditions, various techniques have been developed to date.

US Patent No. 6,274,121 discloses superparamagnetic nanoparticles, including metal such as iron oxide, and specifically, nanoparticles having inorganic material attached to the surface thereof. The inorganic material includes a binding site to be coupled with a tissue-specific binding material, or diagnostic or pharmaceutically active material.

US Patent No. 6,638,494 discloses superparamagnetic nanoparticles including a metal such as iron oxide, and also a method of preventing the nanoparticles from aggregating and depositing under the influence of gravity or a magnetic field by attaching a specific carboxylic acid to the surface of the nanoparticles. Such a specific carboxylic acid includes aliphatic dicarboxylic acids, such as maleic acid, tartaric acid, glucaric acid, etc., or aliphatic polycarboxylic acids, such as citric acid, cyclohexane-tricarboxylic acid, etc.

US Serial No. 2004/0058457 discloses functional nanoparticles enclosed by a monolayer, in which the monolayer has a bifunctional peptide attached thereto, and the peptide contains various biopolymers, including DNA and RNA, bound thereto.

GB Patent No. 0223127 discloses a method of encapsulating magnetic nanoparticles with apoferritin proteins.

US Serial No. 2003/0190471 discloses a method of forming manganese zinc oxide nanoparticles in a bi-micellar vesicle, the magnetic nanoparticles thus formed exhibiting improved properties through heat treatment procedures.

US Patent Nos. 4,795,998 and 4,554,098 and US Serial No. 2003/0092029 disclose metal oxide nanoparticles obtained by reducing transition metal ions in a polymer and a method of binding a bioactive component to the polymeric surface of the nanoparticles thus synthesized.

US Patent Nos. 4,452,773 and 5,262,176 and US Serial No. 2003/0185757 disclose magnetic metal oxide nanoparticles coated with a polysaccharide such as dextran.

US Serial No. 2005/0130167 discloses the synthesis of water-soluble magnetic nanoparticles coated with 16-mercaptohexadecanoic acid and the detection of viruses and mRNA in laboratory rats via magnetic labeling using magnetic particle conjugated with transfaction agent, TAT peptide.

Korean Patent Application No. 10-1998-0705262 discloses particles comprising superparamagnetic iron oxide core particles having a starch coating and a polyalkylene oxide coating, and an MRI contrast agent including the same.

However, the water-soluble nanoparticles synthesized using the above methods have the following disadvantages. According to the techniques disclosed in US Patent Nos. 6,274,121 and 6,638,494, US Serial Nos. 2004/0058457, 2003/0190471, and 2005/0130167, US Patent Nos. 6,274,121 and 6,638,494, US Serial Nos. 2004/0058457 and 2003/0190471, GB Patent No. 0223127, and Korean Patent Application No. 10-1998-0705262, the nanoparticles are synthesized in an aqueous solution. In such cases, it is difficult to control the size of the nanoparticles, the synthesized nanoparticles have a nonuniform size distribution, the crystallinity of the nanoparticles is low due to the low-temperature synthesis, and a non-stoichiometric compound is formed.

However, since the size, shape, size distribution, crystallinity, and stoichiometry of the nanoparticles greatly affect the electrical/optical/magnetic/catalytic properties thereof, the magnetic nanoparticles developed to date have exhibited weak magnetic properties, and the metal oxide nanoparticles have exhibited poor electrical/optical properties. Moreover, their low colloidal stability in aqueous solution leads aggregation and non-selective binding in biological applications.

When the magnetic nanoparticles having such problems are used as critical materials in electronic information technology and biotechnology, they show limitations. For example, the nanoparticles developed to date have low biomaterial separation efficiency, low signal amplification effects as the diagnostic probe of MRI, low sensitivity and high noise (non-selective reaction) in a giant magnetoresistive sensor and a micro-hydraulic system biosensor, and low therapeutic effects in drug/gene delivery and magnetic hyperthermia. Furthermore, the metal oxide nanoparticles manifest low catalytic activity.

In order to solve such problems, methods of developing magnetic or metal oxide nanoparticles in an organic solvent at a high temperature have been proposed. In Korean Unexamined Patent Publication Nos. 2003-0082395 and 2003-008234, US Serial No. 2004/0247503, US Patent Nos. 6,262,129 and 6,262,129, US Chemical Transaction (J. Am. Chem. Soc. 2002, vol.124, p8204), and US Material Chemical Transaction (Chem. Matter. 2004, vol 16, p3931), methods of synthesizing magnetic or metal oxide nanoparticles having good quality have been disclosed. The nanoparticles thus synthesized not only are easily controlled their size and shape, which greatly affect the electrical/optical/magnetic properties thereof, but also have high crystallinity and controlled stoichiometry. Although the nanoparticles synthesized using the high-temperature organic phase synthesis method are coated with hydrophobic ligands and are thus efficiently dispersed in the organic solvent, they do not disperse in aqueous solution, and therefore are almost impossible to use in biomedical applications.

Hence, it is needed to make metal oxide nanoparticles having good quality, which are synthesized in an organic solvent at high temperature, stable in an aqueous solution.

US Serial No. 2005/0165120 discloses a method of transferring metal nanoparticles, such as gold, platinum or nickel, dissolved in an organic solvent, to an aqueous solution phase using a cation and anion phase-transfer monomeric ligand (e.g., didodecyldimethylammonium bromide, cetyltrimethylammonium bromide). However, the above technique is problematic because it can be applied only to semiconductors and metal nanoparticles.

WO2002/041826 discloses a method of transferring gold, platinum, and metal oxide nanoparticles, dissolved in an organic solvent, to an aqueous solution phase using a phase-transfer monomeric ligand composed of X-Y-Z (e.g., dimethylaminopyridine, mercaptoundecanoic acid). However, the application thereof is limited only to gold and platinum nanoparticles, and it is difficult to generally apply various nanoparticles therewith. Further, such water-soluble nanoparticles suffer because the phase transfer ligand is easily separated from the nanoparticles in the aqueous solution, and thus aqueous solution-phase colloidal stability, which is essential in applications such as sensing, separation and diagnosis, is remarkably decreased.

Although the present inventors have filed techniques for synthesizing magnetic, semiconductor, and metal oxide nanoparticles coated with multifunctional ligands composed of adhesive region (L_{I})-crosslinking region (L_{II})-reactive region (L_{III}) (Korean Patent Nos. 652251 and 604976), there have been cases in which the crosslinking region and the reactive region are not required, depending on the end uses of the nanoparticles.

### Disclosure of the Invention

Accordingly, in order to provide metal oxide nanoparticles that are stable in an aqueous solution while maintaining intrinsic electrical/optical/magnetic properties, the present inventors have found that water-soluble water-soluble metal oxide nanoparticles can be prepared from water-insoluble metal oxide nanoparticles synthesized in a high-temperature organic phase through phase transfer using phase transfer ligands composed of an adhesive region, an adhesive region-crosslinking region, or an adhesive region-reactive region. Such water-soluble water-soluble metal oxide nanoparticles can be stably dissolved and dispersed in an aqueous solution having a broad pH range and salt concentration.

An embodiment of the present invention provides water-soluble water-soluble metal oxide nanoparticles, characterized in that the metal oxide nanoparticles are coated with phase transfer ligands having an adhesive region, an adhesive region-crosslinking region, or an adhesive region-reactive region.

Another embodiment of the present invention provides a method of preparing water-soluble water-soluble metal oxide nanoparticles, comprising the steps of (1) synthesizing water-insoluble metal oxide nanoparticles in an organic solvent; (2) dissolving the water-insoluble metal oxide nanoparticles in a first solvent and dissolving a phase transfer ligand in a second solvent; and (3) mixing the two solutions achieved in step (2) to thus exchange the surface of the water-insoluble metal oxide nanoparticles with the phase transfer ligand.

A further embodiment of the present invention provides a method of detecting and separating biomaterial and chemical material using the water-soluble metal oxide nanoparticles.

Still a further embodiment of the present invention provides a diagnosis method using the water-soluble metal oxide nanoparticles.

Yet another embodiment of the present invention provides a treatment method using the water-soluble metal oxide nanoparticles.

Still another embodiment of the present invention provides a method of decomposing microorganisms and contaminants using the photoelectron catalytic activity of the water-soluble metal oxide nanoparticles.

### Brief Description of the Drawings

FIG. 1 shows a process of preparing water-soluble magnetic or metal oxide nanoparticles from water-insoluble magnetic or metal oxide nanoparticles through phase transfer;
FIG. 2 shows transmission electron microscopy images of water-insoluble iron oxide, manganese ferrite, cobalt ferrite, nickel ferrite, zinc ferrite, and zinc-manganese ferrite nanoparticles, all having a particle size of 12 nm;
FIG. 3 shows the results of an analysis of the solubility of the water-soluble iron oxide nanoparticles coated with various phase transfer ligands according to the present invention;
FIG. 4 shows the results of an analysis of the stability of the water-soluble iron oxide nanoparticles coated with various phase transfer ligands according to the present invention, depending on changes in salt concentration and pH;
FIG. 5 shows the results of an analysis of the solubility of the water-soluble manganese ferrite nanoparticles coated with various phase transfer ligands according to the present invention;
FIG. 6 shows the results of an analysis of the stability of the water-soluble manganese ferrite nanoparticles coated with various phase transfer ligands according to the present invention, depending on changes in salt concentration and pH;
FIG. 7 shows the results of an analysis of the solubility of the water-soluble cobalt ferrite nanoparticles coated with various phase transfer ligands according to the present invention;
FIG. 8 shows the results of an analysis of the stability of the water-soluble cobalt ferrite nanoparticles coated with various phase transfer ligands according to the present invention, depending on changes in salt concentration and pH;
FIG. 9 shows the results of an analysis of the solubility of the water-soluble nickel ferrite nanoparticles coated with various phase transfer ligands according to the present invention;
FIG. 10 shows the results of an analysis of the stability of the water-soluble nickel ferrite nanoparticles coated with various phase transfer ligands according to the present invention, depending on changes in salt concentration and pH;
FIG. 11 shows the results of an analysis of the solubility of the water-soluble zinc ferrite nanoparticles coated with various phase transfer ligands according to the present invention;
FIG. 12 shows the results of an analysis of the stability of the water-soluble zinc ferrite nanoparticles coated with various phase transfer ligands according to the present invention, depending on changes in salt concentration and pH;
FIG. 13 shows the results of an analysis of the solubility of the water-soluble zinc-manganese ferrite nanoparticles coated with various phase transfer ligands according to the present invention;
FIG. 14 shows the results of an analysis of the stability of the water-soluble zinc-manganese ferrite nanoparticles coated with various phase transfer ligands according to the present invention, depending on changes in salt concentration and pH;
FIG. 15 is an transmission electron microscopy image showing water-insoluble manganese oxide nanoparticles;
FIG. 16 shows the results of an analysis of the solubility of the water-soluble manganese oxide nanoparticles coated with various phase transfer ligands according to the present invention;
FIG. 17 shows the results of an analysis of the stability of the water-soluble manganese oxide nanoparticles coated with various phase transfer ligands according to the present invention, depending on changes in salt concentration and pH;
FIG. 18 is an transmission electron microscopy image showing water-insoluble titanium dioxide nanoparticles;
FIG. 19A is an transmission electron microscopy image showing the water-soluble titanium dioxide nanoparticles coated with various phase transfer ligands according to the present invention;
FIG. 19B shows the UV-Vis absorption spectrum of the water-soluble titanium dioxide nanoparticles coated with various phase transfer ligands according to the present invention;
FIG. 20 shows the results of an analysis of the solubility of the water-soluble titanium dioxide nanoparticles coated with various phase transfer ligands according to the present invention;
FIG. 21 shows the results of an analysis of the stability of the water-soluble titanium dioxide nanoparticles coated with various phase transfer ligands according to the present invention, depending on changes in salt concentration and pH;
FIG. 22 is an transmission electron microscopy image showing water-insoluble iron-platinum alloy nanoparticles;
FIG. 23 shows the results of an analysis of the solubility of the water-soluble iron-platinum alloy nanoparticles coated with various phase transfer ligands according to the present invention;
FIG. 24 shows the results of an analysis of the stability of the water-soluble iron-platinum alloy nanoparticles coated with various phase transfer ligands according to the present invention, depending on changes in salt concentration and pH;
FIG. 25 is an transmission electron microscopy image showing water-insoluble FePt-Au magnetic multicomponent hybrid structure nanoparticles;
FIG. 26A shows the results of an analysis of the solubility of the water-soluble FePt-Au multicomponent hybrid structure nanoparticles coated with the phase transfer ligand according to the present invention;
FIG. 26B shows the results of an analysis of the aqueous solution-phase colloidal stability of the water-soluble FePt-Au multicomponent hybrid structure nanoparticles coated with the phase transfer ligand according to the present invention, depending on changes in salt concentration and pH;
FIG. 27 is an electron microscopy image showing water-insoluble Co@Pt-Au magnetic multicomponent hybrid structure nanoparticles;
FIG. 28A shows the results of an analysis of the solubility of the water-soluble Co@Pt-Au multicomponent hybrid structure nanoparticles coated with the phase transfer ligand;
FIG. 28B shows the results of an analysis of the aqueous solution-phase colloidal stability of the water-soluble Co@Pt-Au multicomponent hybrid structure nanoparticles coated with the phase transfer ligand, depending on changes in salt concentration and pH;
FIG. 29 shows the results of electrophoresis of the iron oxide nanoparticles coated with the phase transfer ligand (BSA) of the present invention and the iron oxide nanoparticles coated with the phase transfer ligand (BSA) of the present invention and having neutravidin bound thereto;
FIG. 30 shows the results of MRI signal increase effects on the iron oxide, manganese ferrite, nickel ferrite, and cobalt ferrite nanoparticles coated with the phase transfer ligand (BSA) of the present invention;
FIG. 31 shows the results of MRI signal increase effects on aggregates of the iron oxide nanoparticles coated with the phase transfer ligand (BSA) of the present invention and having neutravidin, which induced by biotin-coated silica, compared to those of the iron oxide nanoparticles without the biotin-coated silica;
FIG. 32 is a fluorescence spectrum showing the results of the separation of a fluorescent material, which is bound with the phase transfer binder (BSA)-coated iron oxide nanoparticles, from a mixture including another fluorescent material not bound with the nanoparticles, using an external magnetic field; and
FIG. 33 shows the cancer cells-killing effect of the titanium dioxide nanoparticles coated with the phase transfer ligand (DMSA) of the present invention under the radiation of UV light.

### Best Mode for Carrying Out the Invention

In the following detailed description and definitions, only metal oxide nanoparticles fall within the scope of the claimed invention.

In the present disclosure the term "magnetic or metal oxide nanoparticles" means particulate material comprising a magnetic material, a metal oxide, a magnetic alloy or a multicomponent hybrid structure having a diameter of 1∼1000 nm, and preferably, 2∼100 nm. These particles may be exemplified as follows.

Examples of the magnetic material include transition metals including Mn, Fe, Co, Ni, and Nb; lanthanide or actinide metals including Gd, Tb, Dy, Ho, and Sm; MₓO_{y}(M = a trans metal element including Cr, Mn, Fe, Co, Ni, Nb, and Mo, or a lanthanide or actinide element including Sm, Eu, Gd, Tb, Dy, and Ho; 0<x≤20, 0<y≤20); and M^{a}ₓM^{b}_{y}O_{z} (M^{a}= one or more metals selected from among transition metal elements including Cr, Mn, Fe, Co, Ni, and Nb, and lanthanide and actinide elements including Gd, Tb, Dy, Ho, and Sm; M^{b} = one or more metals selected from the group consisting of Group 1 and 2 metal elements including Li, Na, Be, Ca, Ge, Ba, Mg, Sr, and Ra, transition metal elements including Ti, Cu, Zn, Y, Ta, V, Cd, Zr, Mo, Pt, Pd, Rh, Ru, Ag, Ir, Os, Re, W, La, Hf, and Au, lanthanide or actinide elements including Eu, Er, Tm, Yb, Lu, La, Ce, Pr, and Pm, Group 13 elements including B, Al, Ga, and In, Group 14 elements including C, Si, Ge, Sn, and Pb, Group 15 elements including As, Sb, and Bi, and Group 16 elements including S, Se, and Te; 0<x≤20, 0<y≤20, 0<z≤20).

Examples of the metal oxide include MₓO_{y} (Mₓ = one or more metals selected from the group consisting of Group 1 and 2 elements including Li, Na, Be, Ca, Ge, Ba, Mg, Sr, and Ra, transition metal elements including Sc, Ti, V, Y, Tc, Ru, Rh, Pd, Ag, Cd, Hf, Ta, W, Re, Os, Ir, Pt, and Au, Group 13 elements including Al, Ga and In, Group 14 elements including Sn and Pb, Group 15 elements including Bi, and lanthanide or actinide elements including Ce, Pr, Nd, Pm, Er, Tm, Yb, Lu, La, Th, Pa, U, Am, Cm, Bk, Cf, Ex, Fm, Md, No, and Lr; 0<x≤20, 0<y≤20).

Examples of the magnetic alloy include MₓM_{y} (Mₓ = one or more elements selected from among transition metal elements including Cr, Mn, Fe, Co, Ni, Cu, Nb, and Mo, and lanthanide or actinide elements including Gd, Tb, Dy, Ho, and Sm; M_{y} = one or more elements selected from among Group 1 and 2 metal elements including Li, Na, Be, Ca, Ge, Ba, Mg, Sr, and Ra, transition metal elements including Sc, Ti, V, Sr, Tc, Rh, Ru, Pd, Ag, Cd, La, Hf, Ta, W, Os, Ir, Pt, and Au, Group 13 elements including B, Al, Ga, and In, Group 14 elements including C, Si, Ge, and Sn, Group 15 elements including P, As, Sb, and Bi, Group 16 elements including S, Te, and Se, and lanthanide or actinide elements including Ce, Pr, Nb, Pm, Eu, Er, Tm, Yb, Lu, Th, Pa, U, Np, Pu, Am, Cm, Bk, Cf, Ex, Fm, Md, No, and Lr; 0<x≤20, 0<y≤20).

The term "multicomponent hybrid structure" means a structure that comprises two or more types of nanoparticles selected from the group consisting of the magnetic material, the metal oxide, and the magnetic alloy as defined above, or that comprises one type of nanoparticle selected from the group consisting of the magnetic material, the metal oxide, and the magnetic alloy as defined above and one or more types of nanoparticles selected from the group consisting of semiconductors (composed of two or more elements selected from among Group 13 elements including Ga, In, and Tl, Group 12 elements including Zn, Cd and Hg, Group 14 elements including C, Si, Ge, Sn, and Pb, Group 15 elements including N, P, As, Sb, and Bi, and Group 16 elements including O, S, Se, Te, and Po), transition metals including Au, Ag, Pt, Cu, Pd, Mo, Os, W, Ti, V, Cr, Zn, Nb, Ru, Rh, and Ir, Group 13 materials including Al, Ga, and In, Group 14 materials including C, Si, Sn, and Pb, Group 15 materials including As, Sb, and Bi, and Group 16 materials including Se, Te, and Po.

In the present disclosure, the term "water-insoluble magnetic or metal oxide nanoparticles" means nanoparticles coated with a hydrophobic surface stabilizer. Typically, since the nanoparticles are prepared using a high-temperature organic solvent-phase synthesis method and coated with a hydrophobic surface stabilizer, they are water-insoluble. The surface stabilizer is an organic functional molecule that is able to stabilize the state and size of the magnetic or metal oxide nanoparticles, a typical example thereof being a surfactant.

In the present disclosure, the term "water-soluble magnetic or water-soluble metal oxide nanoparticles" means nanoparticles characterized in that a layer of hydrophobic surface stabilizer, applied on the water-insoluble magnetic or metal oxide nanoparticles, is exchanged with a layer of hydrophilic phase transfer ligand according to the present disclosure through phase transfer so that the nanoparticles are coated with the hydrophilic phase transfer ligand and thus can be stably dissolved and dispersed in an aqueous solution.

In the present invention, the conversion of the water-insoluble nanoparticles into the water-soluble nanoparticles is achieved by the phase transfer ligand. In the present invention, the phase transfer ligand is composed of an adhesive region L₁ alone, an adhesive region-crosslinking region L_{II} (L_{I}-L_{II}), or an adhesive region-reactive region L_{III} (L_{I}-L_{III}). Below, the individual regions of the phase transfer ligand are specifically described.

The term "adhesive region" means the portion of the phase transfer ligand that has a functional group for attachment to the nanoparticles. In the water-soluble water-soluble metal oxide nanoparticles of the present invention, the coating of the metal oxide nanoparticles with the phase transfer ligand is realized through a chemical bond between the nanoparticles and the adhesive region. Such a chemical bond is not particularly limited, and includes, for example, a coordinate bond, a covalent bond, a hydrogen bond, a Van der Waals bond, or an ionic bond. Thus, the adhesive region preferably has a functional group which has high affinity to material constituting the nanoparticles, and may be variously selected depending on the type of material constituting the nanoparticles. The adhesive region may have a functional group selected from the group consisting of -COOH, -NH₂, -SH, -SS-, -CONH₂, -PO₃H, -PO₄H₂, -PO₂(OR₁)(OR₂) (R₁, R₂ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), SO₃H, -SO₄H, -NO₂, -CHO, -COSH, -COX, -COOCO-,-CORCO- (R= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -COOR, -CONH-, -CN, -NROH (R = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁NR₂R₃(R₁, R₂, R₃ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -CONHNR₁R₂ (R₁, R₂= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁R₂R₃X (R₁, R₂, R₃ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), and -OH. The adhesive region is essentially included in the phase transfer ligand, and thus the phase transfer ligand may be composed solely of the adhesive region as mentioned above.

The term "crosslinking region" means the portion of the phase transfer ligand having a functional group which is to be crosslinked with a phase transfer ligand adjacent thereto. As such, the term "crosslinking" indicates a bond between one phase transfer ligand and another phase transfer ligand adjacent thereto through intermolecular interaction. The intermolecular interaction (e.g., a hydrogen bond, a covalent bond (e.g., a disulfide bond), an ionic bond, a coordinate bond) is not particularly limited, and thus the crosslinkable functional group may be variously selected depending on the type of intermolecular interaction. The crosslinking region contains a functional group, for example, -COOH, -NH₂, -SH, -CONH₂, -PO₃H, -PO₄H₂, -PO₂(OR₁)(OR₂) (R₁, R₂ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), SO₃H, -SO₄H, -NO₂, -CHO, -COSH, -COX, -COOR, -CONH-, -CN,-NROH (R = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁NR₂R₃ (R₁, R₂, R₃= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -CONHNR₁R₂ (R₁, R₂= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁R₂R₃X (R₁, R₂, R₃ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -OH, -SCOCH₃, -F,-Cl, -Br, -I, -SCN, -NCO, -OCN, -epoxy, -hydrazone, -alkene, or -alkyne. Although the crosslinking region may be included in the phase transfer ligand depending on need (e.g., increase in stability), it may be excluded in the case where the metal oxide nanoparticles are stable in an aqueous solution even without the crosslinking region.

The term "reactive region" means the portion of the phase transfer ligand having a functional group which is to be attached to an active component, and preferably means the end portion of the ligand opposite the adhesive region. The functional group of the reactive region may vary depending on the type and chemical formula of the active component (Table 1). In the present invention, the reactive region includes, for example, but is not limited to, -COOH, -NH₂, -SH, -CONH₂, -PO₃H, -PO₄H₂, -PO₂(OR₁)(OR₂) (R₁, R₂ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), SO₃H, -SO₄H, -NO₂, -CHO, -COSH, -COX, -COOR, -CONH-, -CN, -NROH (R= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁NR₂R₃ (R₁, R₂, R₃= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -CONHNR₁R₂ (R₁, R₂= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁R₂R₃X (R₁, R₂, R₃ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -OH, -SCOCH₃, -F,-Cl, -Br, -I, -SCN, -NCO, -OCN, -epoxy, -hydrazone, -alkene or -alkyne. Although the reactive region may be included in the phase transfer ligand depending on need (e.g., binding to other bioactive materials and chemically active materials), it may be excluded in the case where the usage of the water-soluble nanoparticles need not be bound to the other materials.

**TABLE 1**

| Examples of Functional Groups of Reactive Region in Phase Transfer Ligand | | |
|---|---|---|
| **I** | **II** | **III** |
| R-NH₂ | R'-COOH | R-NHCO-R' |
| R-SH | R'-SH | R-SS-R |
| R-OH | R'-(Epoxy) | R-OCH₂C(OH)CH₂-R' |
| RH-NH₂ | R'-(Epoxy) | R-NHCH₂C(OH)CH₂-R' |
| R-SH | R'-(Epoxy) | R-SCH₂C(OH)CH₂-R' |
| R-NH₂ | R'-COH | R-N=CH-R' |
| R-NH₂ | R'-NCO | R-NHCONH-R' |
| R-NH₂ | R'-NCS | R-NHCSNH-R' |
| R-SH | R'-COCH₂ | R'-COCH₂S-R |
| R-SH | R'-O(C=O)X | R-OCH₂(C=O)O-R' |
| R-(Aziridine) | R'-SH | R-CH₂CH(NH₂)CH₂S-R' |
| R-CH=CH₂ | R'-SH | R-CH₂CHS-R' |
| R-OH | R'-NCO | R'-NHCOO-R |
| R-SH | R'-COCH₂X | R-SCH₂CO-R' |
| R-NH₂ | R'-CON₃ | R-NHCO-R' |
| R-COOH | R'-COOH | R-(C=O)O(C=O)-R' + H₂O |
| R-SH | R'-X | R-S-R' |
| R-NH₂ | R'CH₂C(NH²⁺)OCH₃ | R-NHC(NH²⁺)CH₂-R' |
| R-OP(O²⁻)OH | R'-NH₂ | R-OP(O²⁻)-NH-R' |
| R-CONHNH₂ | R'-COH | R-CONHN=CH-R' |
| R-NH₂ | R'-SH | R-NHCO(CH₂)₂SS-R' |

| | | |
|---|---|---|
| (I: functional group of the reactive region of the phase transfer ligand, II: active component, III: the reaction examples between I and II bound to each other) | | |

In the preparation of the water-soluble nanoparticles according to the present invention, a compound that intrinsically has a functional group necessary for the adhesive region, the crosslinking region, or the reactive region may be used as the phase transfer ligand, or otherwise a compound that is changed or prepared to have the above functional group through a chemical reaction known in the art may be used as the phase transfer ligand.

In general, one example of a phase transfer ligand is a protein. The protein is a polymer composed of many more amino acids than peptide molecules, that is, hundreds to hundreds of thousands of amino acids, contains -COOH and -NH₂ functional groups at both ends thereof, and includes tens of -COOH, -NH₂, -SH, -OH, and -CONH₂ groups. Thereby, the protein naturally includes the adhesive region and the reactive region (L_{I}-L_{III}) or the adhesive region and the crosslinking region (L_{I}-L_{II}), and may be efficiently used as the phase transfer ligand. Typical examples of a protein as phase transfer ligand include structural proteins, storage proteins, transport proteins, hormone proteins, receptor proteins, contractile proteins, defense proteins, and enzyme proteins. More specifically, the protein is exemplified by albumins, antibodies, secondary antibodies, antigens, hemoglobin, streptavidin, myosin, cytochrome, glycinin, heparin, avidin, protein A, protein G, protein S, immunoglobulin, lectin, selectin, angiopoietin, anticancer proteins, antibiotic proteins, hormone antagonistic proteins, interleukin, interferon, growth factor proteins, tumor necrosis factor proteins, endotoxin proteins, lymphotoxin proteins, tissue plasminogen activators, urokinase, streptokinase, protease inhibitors, alkyl phosphocholine, surfactants, cardiovascular pharmaceuticals, neuro pharmaceuticals, and gastrointestinal pharmaceuticals.

In general, another example of a phase transfer ligand is a lipid. The lipid is a biomaterial having both a hydrophobic portion and a hydrophilic portion, and the surface of hydrophobic nanoparticles is coated through hydrophobic attraction to thus stably disperse the magnetic and metal oxide nanoparticles in an aqueous solution. Typical examples of a lipid as phase transfer ligand include fatty acids, steroids, terpenoids, phospholipids, glycolipids, and fatty proteins, and the lipid is more specifically exemplified by simple lipids or complex lipids having -PO₄H, -SH, -NH₂, -COOH, -COOR, - SO₂OR, or -OH at the end thereof.

In the present invention, an embodiment of the preferred phase transfer ligand is sugar. Sugar includes monosaccharides, disaccharides, oligosaccharides, and polysaccharides. The sugar contains many -OH functional groups, and thus may be used as the adhesive region, the crosslinking region, and the reactive region. Further, the -OH functional group may be easily substituted with another functional group (e.g., -SH, -NH₂) through a chemical reaction, and consequently, the sugar may be used as the phase transfer ligand. Typical examples of sugar preferred in the present invention include glucose, mannose, fucose, N-acetylglucomin, N-acetylgalactosamin, N-acetylneuramic acid, fructose, levulose, xylose, sorbitol, sucrose, maltose, glycoaldehyde, dihydroxyacetone, erythrose, erythrulose, arabinose, xylulose, fractose, lactose, trehalose, melibiose, cellobiose, raffinose, melezitose, maltoriose, stachyose, schrodose, xylan, araban, hesoxan, fructan, galactan, mannan, agaropectin, alginic acid, carrageenan, hemicellulose, dextran, carbodextran, starch, hypromellose, cellulose, amylose, dioxyacetone, glycerinaldehyde, chitin, agarose, dextrin, ribose, riburose, galactose, carboxymethylcellulose, and glycogen. Still a further example of a phase transfer ligand is a peptide. Peptides, which are oligomers composed of single amino acids, contain-COOH and -NH₂ functional groups at both ends thereof and thus naturally have an adhesive region and a reactive region (L_{I}-L_{III}), and are consequently able to serve as the phase transfer ligand. Further, some amino acids have -SH, - COOH, -NH₂, -CONH₂, -NHCNH₂(N⁺H₂), -CNHCH(N⁺H₂)CH- or -OH groups at the side chain thereof, and a peptide having such an amino acid may be used as the phase transfer ligand composed of an adhesive region and a crosslinking region (L_{I}-L_{II})·
Still another example of a phase transfer ligand is a nucleic acid. Since nucleic acids, which are polymers composed of a plurality of nucleotides, contain -PO₄H and -OH functional groups at both ends thereof, they are naturally provided with the adhesive region and the reactive region (L_{I}-L_{III}) or the adhesive region and the crosslinking region (L_{I}-L_{II}), and thus may be used as the phase transfer ligand. Furthermore, a nucleic acid may be appropriately changed to have a functional group, such as -PO₄H, -SH, -NH₂, -COOH, -OH, at the 3'-end, the 5'-end, or side chain thereof, if necessary.

Another embodiment of the preferred phase transfer ligand in the present invention is a chemical monomer. The chemical monomer contains, at the end or side chain thereof, a functional group of -COOH, -NH₂, -SH, -SS-, -CONH₂, -PO₃H, -PO₄H₂,-PO₂(OR₁)(OR₂) (R₁, R₂ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), SO₃H, -SO₄H, -NO₂, -CHO, -COSH, -COX,-COOCO-, -CORCO- (R= C₁Hₘ, 0≤l≤3, 0≤m≤2l+1), -COOR, -CN, -N₃, -N₂, -NROH (R= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁NR₂R₃ (R₁, R₂, R₃= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -CONHNR₁R₂ (R₁, R₂= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁R₂R₃X (R₁,R₂,R₃ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -OH, -SCOCH₃, -F, -Cl, -Br,-I, -SCN, -NCO, -OCN, -epoxy, -hydrazone, -alkene, and -alkyne.

Preferably tetraalkylammonium halide (NR₄⁺X⁻) (R=CₚH_{q}OᵣNₛSₜ, 0≤p,q,r,s,t≤10, X=F, Cl, Br, I) is used. This compound is a typical example of a phase transfer ligand composed solely of the adhesive region L₁. An alternative is dimercaptosuccinic acid. Dimercaptosuccinic acid has both -SH and -COOH functional groups, and thus may be used as the phase transfer ligand having the adhesive region and the reactive region. In the prior patent applications filed by the present inventors (Korean Patent Application Nos. 2004-0070303 and 2004-0070304), dimercaptosuccinic acid is oxidized, and thereby a crosslinking region is included. However, in the present invention, dimercaptosuccinic acid is used as the phase transfer ligand, in which the crosslinking region is eliminated through the reduction of the disulfide bond thereof. Furthermore, examples of the chemical monomer include, but are not limited to, carboxyalkyl tetraalkylammonium halide (COOH(CH₂)ₙNR₄⁺X⁻) (R=CₚH_{q}OᵣNₛSₜ, 0≤p,q,r,s,t≤10, X=F, Cl, Br, I), carboxyalkyl phosphoric acid (COOH(CH₂)ₙPO₄H)(0≤n≤10), carboxyalkylthiol (COOH(CH₂)ₙSH,0≤n≤10), mercaptosuccinic acid, dimercaptosuccinic acid, mercaptomaleic acid, dimercaptomaleic acid, mercaptopentadionic acid, and dimercaptopentadionic acid.

Still yet another embodiment of the preferred phase transfer ligand in the present invention is a polymer. The polymer has various functional groups constituting the adhesive regions, and may have a crosslinking region and a reactive region depending on the need. Examples of the polymer include polyphosphazene, polyacrylic acid, polylactide, polylactide-co-glycolide, polycaprolactone, polycaprolactam, polyanhydride, polymalic acid and derivatives thereof, polyalkylcyanoacrylate, polyhydroxyalkylate, polycarbonate, polyorthoester, polyethyleneglycol, poly-L-lycine, polyglycolide, polymethylmethacrylate, polyvinylpyrrolidone, and copolymers thereof.

The "active component", which is bound with the reactive region of the phase transfer ligand of the present invention, may be selected depending on the intended use of the water-soluble nanoparticles, and includes, for example, bioactive components, polymers, or inorganic supports.

Examples of the bioactive component include pharmaceutically active substances, such as nucleic acids, peptides, molecular recognition chemical molecules and biomolecules, signal transfer chemical molecules and biomolecules, enzyme chemical molecules and biomolecules, structural control chemical molecules and biomolecules, storage chemical molecules and biomolecules, transport chemical molecules and biomolecules, hormone chemical molecules and biomolecules, decomposition chemical molecules and biomolecules, receptor proteins, contractile proteins, defense proteins, enzyme proteins, gastrointestinal pharmaceuticals, cardiovascular pharmaceuticals, and neuro pharmaceuticals; and more specifically, include antigens, RNA, DNA, hapten, hemoglobin, heparin, avidin, neutravidin, streptavidin, protein A, protein G, lectin, selectin, aptamer, anticancer agents, antibiotic agents, hormones, hormone antagonists, interleukin, interferon, growth factors, tumor necrosis factors, endotoxin, lymphotoxin, tissue plasminogen activators, urokinase, streptokinase, protease inhibitors, alkyl phosphocholine, redox enzymes, decomposition enzymes, isomerization enzymes, synthesis enzymes, enzyme cofactors, and enzyme inhibitors.

Examples of the polymer used as the active component include polyphosphazene, polylactide, polylactide-co-glycolide, polycaprolactone, poly-L-lycine, polycaprolactam, polyanhydride, polycarbonate, polymalic acid and derivatives thereof, polyalkylcyanoacrylate, polyorthoester, polyethyleneglycol, polyhydroxyalkylate, polyglycolide, polymethylmethacrylate, and polyvinylpyrrolidone.

Examples of the inorganic support used as the active component include, but are not limited to, metal oxides (composed of one or more elements selected from among Group 1 and 2 metal elements including Li, Na, Be, Ca, Ge, Ba, Mg, Sr, and Ra, transition metal elements including Sc, Ti, V, Y, Tc, Mn, Fe, Co, Ni, Zn, Zr, Nd, Cr, Ru, Rh, Pd, Ag, Cd, Hf, Ta, W, Re, Os, Ir, Pt, and Au, Group 13 elements including B, Al, Ga and In, Group 14 elements including C, Si, Ge, An and Pb, Group 15 elements including P, As, Sb and Bi, and lanthanide or actinide elements including Ce, Pr, Nd, Pm, Sm, Tb, Dy, Ho, Er, Tm, Yb, Lu, La, Th, Pa, U, Am, Cm, Bk, Cf, Ex, Fm, Md, No, and Lr), semiconductors (composed of two or more elements selected from among Group 13 elements including Ga, In and Tl, Group 12 elements including Zn, Cd, and Hg, Group 14 elements including C, Si, Ge, Sn, and Pb, Group 15 elements including N, P, As, Sb, and Bi, and Group 16 elements including O, S, Se, Te and Po), carbon materials, transition metal materials including Au, Ag, Pt, Cu, Pd, Mo, Os, W, Ti, W, V, Cr, Zn, Nb, Ru, Rh, Ir, Co, Mn, Fe, Ni, Cr, Y, Zr, Cd, Ta, and Re, Group 13 element materials such as Al, Ga, and In, and Group 14 element materials including C, Si, Sn, and Pb.

In addition, according to the present invention, the water-soluble water-soluble metal oxide nanoparticles are prepared using the method of preparing water-soluble magnetic or water-soluble metal oxide nanoparticles comprising the steps of (1) synthesizing water-insoluble metal oxide nanoparticles in an organic solvent; (2) dissolving the water-insoluble metal nanoparticles in a first solvent and dissolving the phase-transfer ligand in a second solvent; and (3) mixing the two solutions achieved in step (2) to thus exchange the surface of the water-insoluble metal oxide nanoparticles with the phase transfer ligand.

In the above-mentioned preparation method, step (1) is a process of preparing the water-insoluble metal oxide nanoparticles. In the present invention, the water-insoluble metal oxide nanoparticles may be prepared in a manner such that a precursor for the nanoparticles is added to an organic solvent at 10∼600°C, including a surface stabilizer, and is then subjected to a chemical reaction while the temperature and time appropriate for the preparation of the desired nanoparticles are maintained, thus growing nanoparticles, after which the nanoparticles thus formed are separated and purified.

As such, examples of the organic solvent include, but are not limited to, benzene solvents (e.g., benzene, toluene, halobenzene, etc.), hydrocarbon solvents (e.g., octane, nonane, decane, etc.), ether solvents (e.g., benzyl ether, phenyl ether, hydrocarbon ether, etc.), polymer solvents, and ionic liquids.

In step (2) of the preparation method, the metal oxide nanoparticles prepared in the previous step are dissolved in the first solvent, and the phase transfer ligand is dissolved in the second solvent. The first solvent is exemplified by benzene solvents (e.g., benzene, toluene, halobenzene, etc.), hydrocarbon solvents (e.g., pentane, hexane, nonane, decane, etc.), ether solvents (e.g., benzyl ether, phenyl ether, hydrocarbon ether, etc.), halohydrocarbons (e.g., methylene chloride, methane bromide, etc.), alcohols (e.g., methanol, ethanol, etc.), sulfoxide solvents (e.g., dimethylsulfoxide, etc.), amide solvents (e.g., dimethylformamide, etc.), and ionic liquids. The second solvent includes water, in addition to the solvent useful as the above-mentioned first solvent.

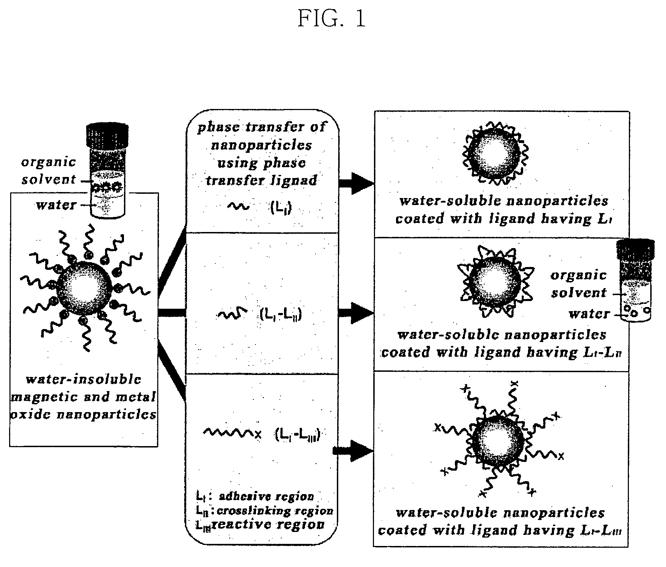

In step (3) of the preparation method, the two solutions are mixed. Here, the organic surface stabilizer of the metal oxide nanoparticles is substituted with the phase transfer ligand, thus producing the water-soluble metal oxide nanoparticles (FIG. 1). The water-soluble water-soluble metal oxide nanoparticles thus produced may be separated using a method known in the art. Typically, since the water-soluble nanoparticles are produced as a precipitate, they are preferably separated through centrifugation or filtration.

Additionally, the metal oxide nanoparticles coated with the phase transfer ligand, separated in step (3), may be added with water or a buffer solution, yielding water-soluble metal oxide nanoparticles which are efficiently dispersed without aggregation under the conditions of a pH of 1∼11 and a salt concentration of about 1 M or less.

The water-soluble metal oxide nanoparticles according to the present invention may be used in various fields, including the separation and detection of materials, the diagnosis and treatment of diseases, and the decomposition of microorganisms and contaminants.

The water-soluble metal oxide nanoparticles according to the present invention may function as a probe material for detecting various biomolecules including proteins, antigens, peptides, DNA, RNA, and viruses, or chemical materials including volatile organic compounds, NOₓ, COₓ, endocrine disruptors (dioxins, DDT, phenols, phthalates, styrene dimers, benzopyrene), and polycyclic aromatic hydrocarbons. As examples thereof, the usable magnetic biosensor includes, but is not limited to, a giant magnetoresistor (GMR), a tunneling magnetoresistor (TMR), a colossal magnetoresistor (CMR), an anisotropic magnetoresistor (AMR), a magnetic force microscope (MFM), a superconducting quantum interference device (SQUID), magnetooptical Kerr effect (MOKE) equipment, a cantilever, a quartz crystal microbalance (QCM), a magnetic resonance imaging (MRI) device, and an electrochemical sensor.

In particular, the water-soluble metal oxide nanoparticles according to the present invention may also be applied to the separation of various biomolecules and chemical materials using the interaction with an external magnetic field, and furthermore, may be applied to magnetic micro-hydraulic sensors to thus separate and detect the biomolecules.

When the water-soluble metal oxide nanoparticles according to the present invention are injected *in vivo,* either conjugated or not conjugated to cancer-specific bioactive material, they change the spin relaxation speed of water molecules therearound to thus alter MR signals in order to improve the diagnostic effects, thereby making it possible to serve as a diagnostic probe for molecular magnetic resonance imaging.

The water-soluble metal oxide nanoparticles according to the present invention may be used for biomedical applications through the delivery of drugs or genes induced by an external magnetic field. The drugs to be delivered include pharmaceutically active components, such as gastrointestinal pharmaceuticals, cardiovascular pharmaceuticals, and neuro pharmaceuticals, and specifically include, but are not limited to, an anticancer agent, an antibiotic agent, a hormone, a hormone antagonist, interleukin, interferon, growth factor, tumor necrosis factor, endotoxin, lymphotoxin, a tissue plasminogen activator, a protease inhibitor, urokinase, streptokinase, and alkyl phosphocholine.

Further, the water-soluble metal oxide nanoparticles according to the present invention may be used for thermotherapy using heat generated by the nanoparticles.

Furthermore, the water-soluble metal oxide nanoparticles according to the present invention may be implanted in the body to replace for the functions of parts of the body.

Moreover, the water-soluble metal oxide nanoparticles according to the present invention may be used for cancer treatment by killing cancer cells using the photoelectron catalytic effects thereof, and may also be used for the decomposition of various microorganisms, and contaminants including polycyclic aromatic hydrocarbons, such as formaldehyde, benzene, toluene, and xylene, volatile organic materials, etc.

Below, the present invention is described in detail through the following examples, which are set forth to illustrate the present invention, which will be apparent by those skilled in the art.

Only those (parts of) examples that refer to metal oxide nanoparticles fall within the claimed invention. Likewise, only examples that employ sugar, monomer or polymer phase transfer ligands as defined earlier fall within the claimed invention. Other examples are comparative. Furthermore, the synthesis of water-soluble iron oxide nanoparticles in Example 12 also does not form part of the invention as claimed and is therefore comparative.

### Comparative Example 1: Synthesis of Water-Soluble Iron Oxide, Manganese Ferrite, Cobalt Ferrite, Nickel Ferrite, Zinc Ferrite, and Zinc-Manganese Ferrite. Nanoparticles Coated with Bovine Serum Albumin (BSA, L_{I}-L_{III})

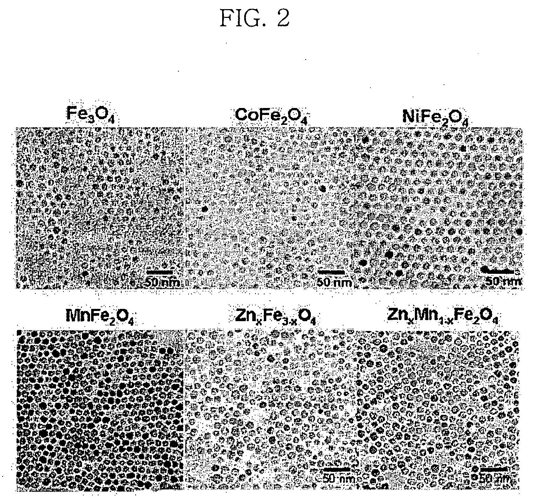

Water-insoluble iron oxide, manganese ferrite, cobalt ferrite, nickel ferrite, zinc ferrite, and zinc-manganese ferrite nanoparticles were synthesized according to the technique disclosed in Korean Patent Application Nos. 2004-0070303 and 2004-0070304, filed by the present inventors. All of the obtained nanoparticles were observed to have a particle size of about 12 nm, as intended, and to have a uniform size distribution and high crystallinity (FIG. 2).

The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). 10 mg of BSA was dissolved in 1 ml of deionized water and was then mixed with the above precipitate, thus synthesizing iron oxide, manganese ferrite, cobalt ferrite, nickel ferrite, zinc ferrite, and zinc-manganese ferrite nanoparticles, individually coated with BSA. Subsequently, using a Sephacryl S-300 column, unreacted BSA was removed, thereby obtaining water-soluble iron oxide, manganese ferrite, cobalt ferrite, nickel ferrite, zinc ferrite, and zinc-manganese ferrite nanoparticles coated with pure BSA.

### Comparative Example 2: Synthesis of Water-Soluble Iron Oxide, Manganese Ferrite, Cobalt Ferrite, Nickel Ferrite, Zinc Ferrite, and Zinc-Manganese Ferrite Nanoparticles Coated with Human Serum Albumin (HSA, L_{I}-L_{III})

As water-insoluble iron oxide, manganese ferrite, cobalt ferrite, nickel ferrite, zinc ferrite, and zinc-manganese ferrite nanoparticles, the same iron oxide, manganese ferrite, cobalt ferrite, nickel ferrite, zinc ferrite, and zinc-manganese ferrite nanoparticles as those of Example 1 were used. The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). 10 mg of HSA was dissolved in 1 ml of deionized water and was then mixed with the above precipitate, thus synthesizing iron oxide, manganese ferrite, cobalt ferrite, nickel ferrite, zinc ferrite, and zinc-manganese ferrite nanoparticles, individually coated with HSA. Subsequently, using a Sephacryl S-300 column, unreacted HSA was removed, thereby obtaining water-soluble iron oxide, manganese ferrite, cobalt ferrite, nickel ferrite, zinc ferrite, and zinc-manganese ferrite nanoparticles coated with pure HSA.

### Comparative Example 3: Synthesis of Water-Soluble Iron Oxide, Manganese Ferrite, Cobalt Ferrite, Nickel Ferrite, Zinc Ferrite, and Zinc-Manganese Ferrite Nanoparticles Coated with Human ImmunoGlobulin G (hIgG, L_{I}-L_{III})

As water-insoluble iron oxide, manganese ferrite, cobalt ferrite, nickel ferrite, zinc ferrite, and zinc-manganese ferrite nanoparticles, the same iron oxide, manganese ferrite, cobalt ferrite, nickel ferrite, zinc ferrite, and zinc-manganese ferrite nanoparticles as those of Example 1 were used. The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). 10 mg of hIgG was dissolved in 1 ml of deionized water and was then mixed with the above precipitate, thus synthesizing iron oxide, manganese ferrite, cobalt ferrite, nickel ferrite, zinc ferrite, and zinc-manganese ferrite nanoparticles, individually coated with hIgG. Subsequently, using a Sephacryl S-300 column, unreacted hIgG was removed, thereby obtaining water-soluble iron oxide, manganese ferrite, cobalt ferrite, nickel ferrite, zinc ferrite, and zinc-manganese ferrite nanoparticles coated with pure hIgG.

### Comparative Example 4: Synthesis of Water-Soluble Iron Oxide, Manganese Ferrite, Cobalt Ferrite, Nickel Ferrite, Zinc Ferrite, and Zinc-Manganese Ferrite Nanoparticles Coated with Neutravidin (Ntv, L_{I}-L_{III})

As water-insoluble iron oxide, manganese ferrite, cobalt ferrite, nickel ferrite, zinc ferrite, and zinc-manganese ferrite nanoparticles, the same iron oxide, manganese ferrite, cobalt ferrite, nickel ferrite, zinc ferrite, and zinc-manganese ferrite nanoparticles as those of Example 1 were used. The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). 10 mg of Ntv was dissolved in 1 ml of deionized water and was then mixed with the above precipitate, thus synthesizing iron oxide, manganese ferrite, cobalt ferrite, nickel ferrite, zinc ferrite, and zinc-manganese ferrite nanoparticles, individually coated with Ntv. Subsequently, using a Sephacryl S-300 column, unreacted Ntv was removed, thereby obtaining water-soluble iron oxide, manganese ferrite, cobalt ferrite, nickel ferrite, zinc ferrite, and zinc-manganese ferrite nanoparticles coated with pure Ntv.

### Comparative Example 5: Synthesis of Water-Soluble Iron Oxide, Manganese Ferrite, Cobalt Ferrite, Nickel Ferrite, Zinc Ferrite, and Zinc-Manganese Ferrite Nanoparticles Coated with Hemoglobin (L_{I}-L_{III})

As water-insoluble iron oxide, manganese ferrite, cobalt ferrite, nickel ferrite, zinc ferrite, and zinc-manganese ferrite nanoparticles, the same iron oxide, manganese ferrite, cobalt ferrite, nickel ferrite, zinc ferrite, and zinc-manganese ferrite nanoparticles as those of Example 1 were used. The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). 10 mg of hemoglobin was dissolved in 1 ml of deionized water and was then mixed with the above precipitate, thus synthesizing iron oxide, manganese ferrite, cobalt ferrite, nickel ferrite, zinc ferrite, and zinc-manganese ferrite nanoparticles, individually coated with hemoglobin. Subsequently, using a Sephacryl S-300 column, unreacted hemoglobin was removed, thereby obtaining water-soluble iron oxide, manganese ferrite, cobalt ferrite, nickel ferrite, zinc ferrite, and zinc-manganese ferrite nanoparticles coated with pure hemoglobin.

### Comparative Example 6: Synthesis of Water-Soluble Iron Oxide, Manganese Ferrite, Cobalt Ferrite, Nickel Ferrite, Zinc Ferrite, and Zinc-Manganese Ferrite Nanoparticles Coated with Heparin (L_{I}-L_{III})

As water-insoluble iron oxide, manganese ferrite, cobalt ferrite, nickel ferrite, zinc ferrite, and zinc-manganese ferrite nanoparticles, the same iron oxide, manganese ferrite, cobalt ferrite, nickel ferrite, zinc ferrite, and zinc-manganese ferrite nanoparticles as those of Example 1 were used. The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). 10 mg of heparin was dissolved in 1 ml of deionized water and was then mixed with the above precipitate, thus synthesizing iron oxide, manganese ferrite, cobalt ferrite, nickel ferrite, zinc ferrite, and zinc-manganese ferrite nanoparticles, individually coated with heparin. Subsequently, using a Sephacryl S-300 column, unreacted heparin was removed, thereby obtaining water-soluble iron oxide, manganese ferrite, cobalt ferrite, nickel ferrite, zinc ferrite, and zinc-manganese ferrite nanoparticles coated with pure heparin.

### Example 7: Synthesis of Water-Soluble Iron Oxide, Manganese Ferrite, Cobalt Ferrite, Nickel Ferrite, Zinc Ferrite, and Zinc-Manganese Ferrite Nanoparticles Coated with Dextran (L_{I}-L_{III})

As water-insoluble iron oxide, manganese ferrite, cobalt ferrite, nickel ferrite, zinc ferrite, and zinc-manganese ferrite nanoparticles, the same iron oxide, manganese ferrite, cobalt ferrite, nickel ferrite, zinc ferrite, and zinc-manganese ferrite nanoparticles as those of Example 1 were used. The water-insoluble nanoparticles (5 mg) were dispersed in 1 ml of 1 M NMe₄OH solved in butanol solution and were then mixed for about 5 minutes, until uniform. Thereafter, a blackish brown precipitate was formed, and this precipitate was separated through centrifugation (2000 rpm, room temperature, 5 min). 10 mg of dextran, having a molecular weight of 10,000 Da, was dissolved in 1 ml of deionized water and was then mixed with the above precipitate, thus synthesizing iron oxide, manganese ferrite, cobalt ferrite, nickel ferrite, zinc ferrite, and zinc-manganese ferrite nanoparticles, individually coated with dextran. Subsequently, using a Sephacryl S-300 column, unreacted dextran was removed, thereby obtaining water-soluble iron oxide, manganese ferrite, cobalt ferrite, nickel ferrite, zinc ferrite, and zinc-manganese ferrite nanoparticles coated with pure dextran.

### Example 8: Synthesis of Water-Soluble Iron Oxide, Manganese Ferrite, Cobalt Ferrite, Nickel Ferrite, Zinc Ferrite, and Zinc-Manganese Ferrite Nanoparticles Coated with Hypromellose (L_{I}-L_{III})

As water-insoluble iron oxide, manganese ferrite, cobalt ferrite, nickel ferrite, zinc ferrite, and zinc-manganese ferrite nanoparticles, the same iron oxide, manganese ferrite, cobalt ferrite, nickel ferrite, zinc ferrite, and zinc-manganese ferrite nanoparticles as those of Example 1 were used. The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). 10 mg of hypromellose, having a molecular weight of 80,000 Da, was dissolved in 1 ml of deionized water and was then mixed with the above precipitate, thus synthesizing iron oxide, manganese ferrite, cobalt ferrite, nickel ferrite, zinc ferrite, and zinc-manganese ferrite nanoparticles, individually coated with hypromellose. Subsequently, using a Sephacryl S-300 column, unreacted hypromellose was removed, thereby obtaining water-soluble iron oxide, manganese ferrite, cobalt ferrite, nickel ferrite, zinc ferrite, and zinc-manganese ferrite nanoparticles coated with pure hypromellose.

### Example 9: Synthesis of Water-Soluble Iron Oxide, Manganese Ferrite, Cobalt Ferrite, Nickel Ferrite, Zinc Ferrite, and Zinc-Manganese Ferrite Nanoparticles Coated with Carboxymethylcellulose (L_{I}-L_{III})

As water-insoluble iron oxide, manganese ferrite, cobalt ferrite, nickel ferrite, zinc ferrite, and zinc-manganese ferrite nanoparticles, the same iron oxide, manganese ferrite, cobalt ferrite, nickel ferrite, zinc ferrite, and zinc-manganese ferrite nanoparticles as those of Example 1 were used. The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). 10 mg of carboxymethylcellulose, having a molecular weight of 90,000 Da, was dissolved in 1 ml of deionized water and was then mixed with the above precipitate, thus synthesizing iron oxide, manganese ferrite, cobalt ferrite, nickel ferrite, zinc ferrite, and zinc-manganese ferrite nanoparticles, individually coated with carboxymethylcellulose. Subsequently, using a Sephacryl S-300 column, unreacted carboxymethylcellulose was removed, thereby obtaining water-soluble iron oxide, manganese ferrite, cobalt ferrite, nickel ferrite, zinc ferrite, and zinc-manganese ferrite nanoparticles coated with pure carboxymethylcellulose.

### Example 10: Synthesis of Water-Soluble Iron Oxide, Manganese Ferrite, Cobalt Ferrite, Nickel Ferrite, Zinc Ferrite, and Zinc-Manganese Ferrite Nanoparticles Coated with Polyvinylalcohol (PVA, L_{I})

As water-insoluble iron oxide, manganese ferrite, cobalt ferrite, nickel ferrite, zinc ferrite, and zinc-manganese ferrite nanoparticles, the same iron oxide, manganese ferrite, cobalt ferrite, nickel ferrite, zinc ferrite, and zinc-manganese ferrite nanoparticles as those of Example 1 were used. The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). 10 mg of PVA, having a molecular weight of 10,000 Da, was dissolved in 1 ml of deionized water and was then mixed with the above precipitate, thus synthesizing iron oxide, manganese ferrite, cobalt ferrite, nickel ferrite, zinc ferrite, and zinc-manganese ferrite nanoparticles, individually coated with PVA. Subsequently, using a Sephacryl S-300 column, unreacted PVA was removed, thereby obtaining water-soluble iron oxide, manganese ferrite, cobalt ferrite, nickel ferrite, zinc ferrite, and zinc-manganese ferrite nanoparticles coated with pure PVA.

### Example 11: Synthesis of Water-Soluble Iron Oxide, Manganese Ferrite, Cobalt Ferrite, Nickel Ferrite, Zinc Ferrite, and Zinc-Manganese Ferrite Nanoparticles Coated with Polyethyleneglycol-Polyacrylic Acid (PAA-PEG, L_{I}-L_{II})

PAA-PEG was prepared through the following procedure. 0.72 g of PAA, having a molecular weight of 2,000 Da, was dissolved in 10 ml of a dichloromethane solution, after which 0.8 g of N-hydroxysuccinimide (NHS) was added thereto. Subsequently, 1.1 g of dicyclohexylcarbodiimide (DCC) was added, and the mixture was allowed to react for 24 hours. The NHS-formulated PAA thus obtained was separated through column chromatography, after which the solvent was removed, thus obtaining a white solid. 0.8 g of the white solid was dissolved in a DMF solution, and 2 g of NH₂-PEG-OH was added thereto, and then the mixture was allowed to react for 24 hours, thus obtaining PAA-PEG in which 50% PEG was substituted.

As water-insoluble iron oxide, manganese ferrite, cobalt ferrite, nickel ferrite, zinc ferrite, and zinc-manganese ferrite nanoparticles, the same iron oxide, manganese ferrite, cobalt ferrite, nickel ferrite, zinc ferrite, and zinc-manganese ferrite nanoparticles as those of Example 1 were used. The water-insoluble nanoparticles (5 mg) were dispersed in a solution (5 mg/ml) of PAA-PEG dissolved in 1 ml of ethanol, and were then mixed for about 10 hours, until uniform. Thereafter, a blackish brown precipitate was formed, and this precipitate was separated through centrifugation (2000 rpm, room temperature, 5 min). The precipitate was dissolved in 1 ml of deionized water, thus synthesizing iron oxide, manganese ferrite, cobalt ferrite, nickel ferrite, zinc ferrite, and zinc-manganese ferrite nanoparticles, individually coated with PAA-PEG. Subsequently, using a Sephacryl S-300 column, unreacted PAA-PEG was removed, thereby obtaining water-soluble iron oxide, manganese ferrite, cobalt ferrite, nickel ferrite, zinc ferrite, and zinc-manganese ferrite nanoparticles coated with pure PAA-PEG.

### Example 12: Synthesis of Water-Soluble Iron Oxide, Manganese Ferrite, Cobalt Ferrite, Nickel Ferrite, Zinc Ferrite, and Zinc-Manganese Ferrite Nanoparticles Coated with Dimercaptosuccinic Acid (DMSA, L_{I}-L_{III}) Without Crosslinking

As water-insoluble iron oxide, manganese ferrite, cobalt ferrite, nickel ferrite, zinc ferrite, and zinc-manganese ferrite nanoparticles, the same iron oxide, manganese ferrite, cobalt ferrite, nickel ferrite, zinc ferrite, and zinc-manganese ferrite nanoparticles as those of Example 1 were used. The obtained water-insoluble nanoparticles (5 mg) were dissolved in 1 ml of toluene, after which the toluene solution was added with 0.5 ml of methanol, in which 20 mg of 2,3-mercaptosuccinic acid (DMSA) was dissolved. After about 24 hours, a blackish brown precipitate was formed, and this precipitate was separated through centrifugation at 2000 rpm at room temperature for 5 min, and was then re-dispersed in 1 ml of deionized water, thus synthesizing iron oxide, manganese ferrite, cobalt ferrite, nickel ferrite, zinc ferrite, and zinc-manganese ferrite nanoparticles, individually coated with DMSA. Subsequently, using a Sephadex G-25 column, unreacted DMSA was removed, thereby obtaining water-soluble iron oxide, manganese ferrite, cobalt ferrite, nickel ferrite, zinc ferrite, and zinc-manganese ferrite nanoparticles coated with pure DMSA.

### Example 13: Synthesis of Water-Soluble Iron Oxide, Manganese Ferrite, Cobalt Ferrite, Nickel Ferrite, Zinc Ferrite, and Zinc-Manganese Ferrite Nanoparticles Coated with Tetramethylammonium Hydroxide (L_{I})

Water-insoluble iron oxide, manganese ferrite, cobalt ferrite, nickel ferrite, zinc ferrite, and zinc-manganese ferrite nanoparticles were synthesized in the same manner as in Example 1. The obtained water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min) and was then re-dispersed in 1 ml of deionized water, thereby obtaining iron oxide, manganese ferrite, cobalt ferrite, nickel ferrite, zinc ferrite, and zinc-manganese ferrite nanoparticles coated with tetramethylammonium hydroxide.

### Example 14: Analysis of Stability in Aqueous Solution of Water-Soluble Iron Oxide, Manganese Ferrite, Cobalt Ferrite, Nickel Ferrite, Zinc Ferrite, and Zinc-Manganese Ferrite Nanoparticles Coated with Phase Transfer Ligands

### a. Analysis of Solubility in Aqueous Solution

The water-insoluble nanoparticles used in Example 1 were dissolved in chloromethane and then had water added. Further, the water-soluble iron oxide, manganese ferrite, cobalt ferrite, nickel ferrite, zinc ferrite, and zinc-manganese ferrite nanoparticles, synthesized in Examples 1 to 13, were dissolved in water and then had hexane added, thus analyzing the change in the solubility of the nanoparticles through a surface substitution reaction.

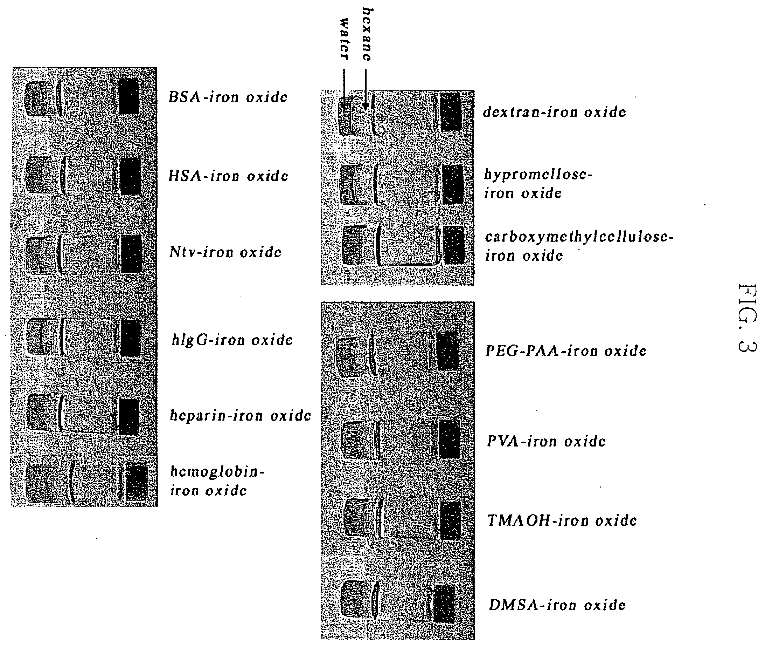

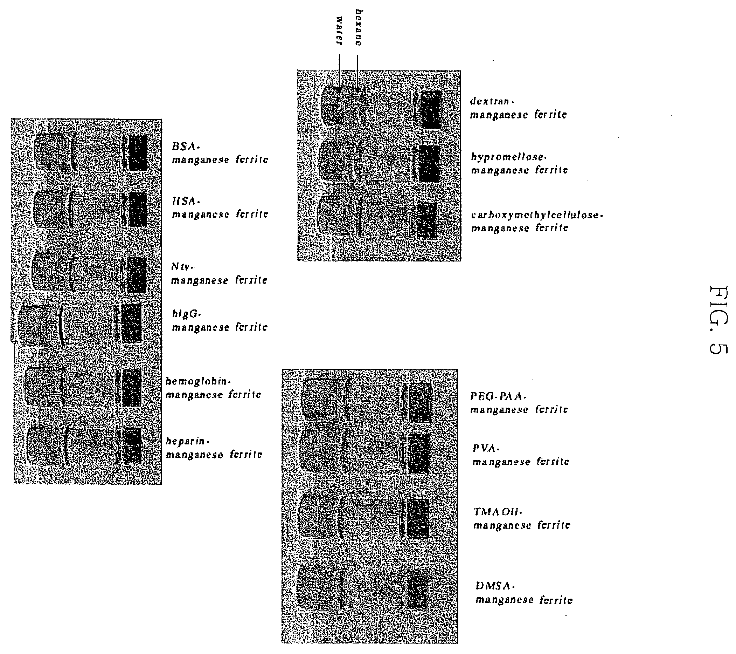

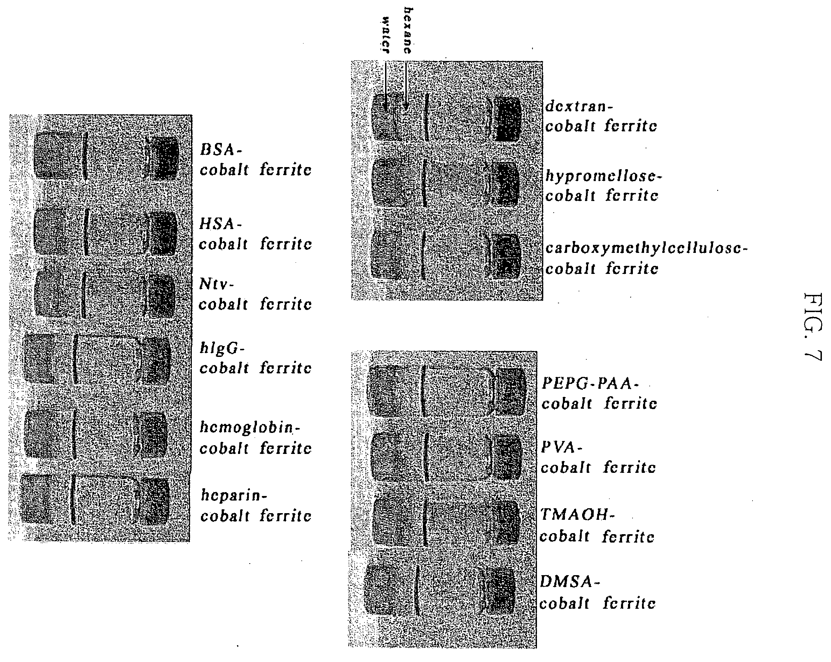

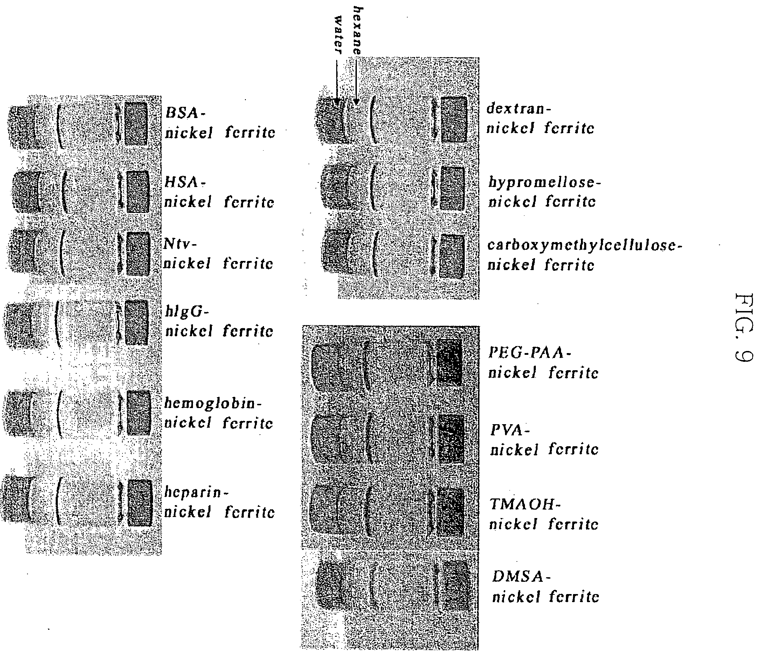

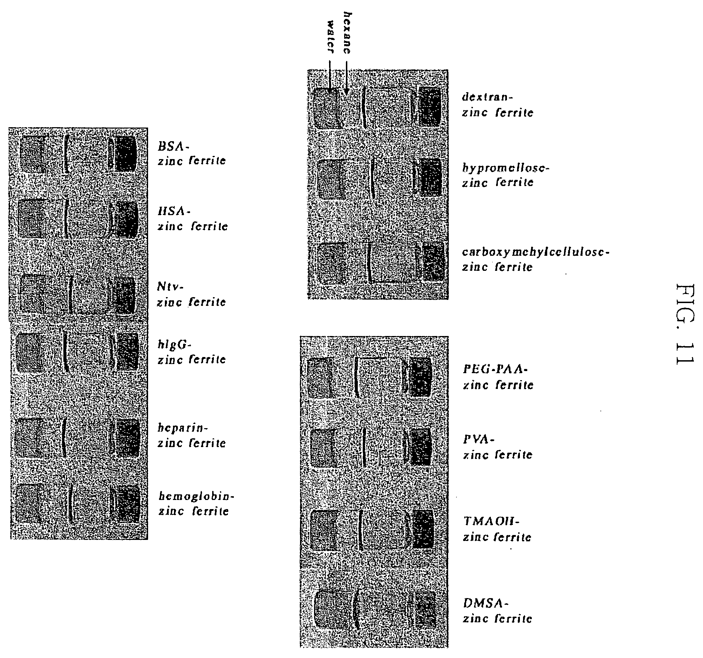

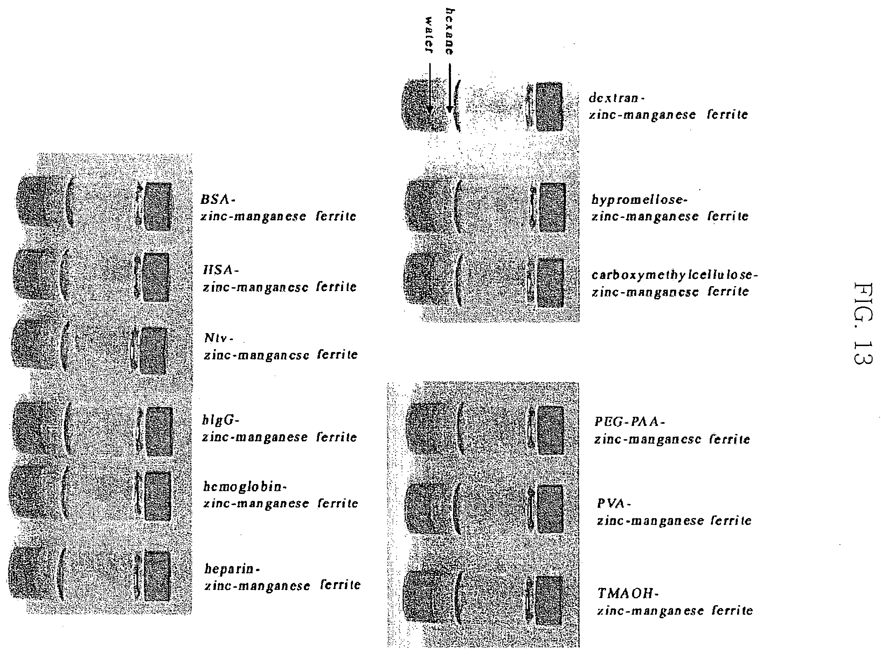

As shown in FIGS. 3, 5, 7, 9, 11, and 13, all of the water-insoluble nanoparticles were determined to be converted into water-soluble nanoparticles. When observed with the naked eye, it could be seen that precipitation or aggregation did not occur, and thus the water-soluble nanoparticles were determined to be well-dispersed in the aqueous solution.

### b. Analysis of Stability depending on Changes in pH and Salt Concentration

The stability of the water-soluble nanoparticles synthesized in Examples 1 to 13 depending on changes in pH and salt concentration was analyzed. In particular, in order to compare the stability of the nanoparticles according to the present invention with the stability of water-soluble nanoparticles obtained through a conventional method, as a control group, gold nanoparticles (WO2002/0041826) coated with dimethylaminopyridine (DMAP) and gold nanoparticles (US2005/0165120) coated with CTAB were used and thus analyzed.

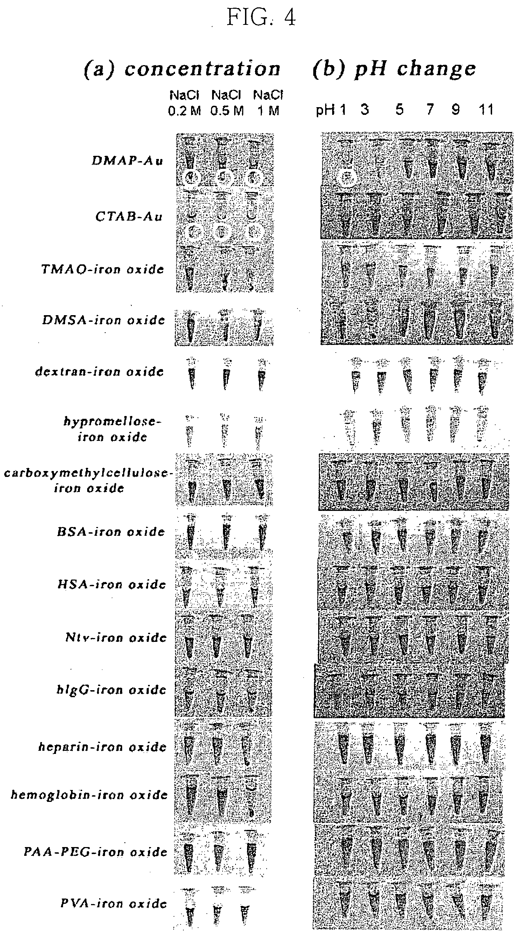

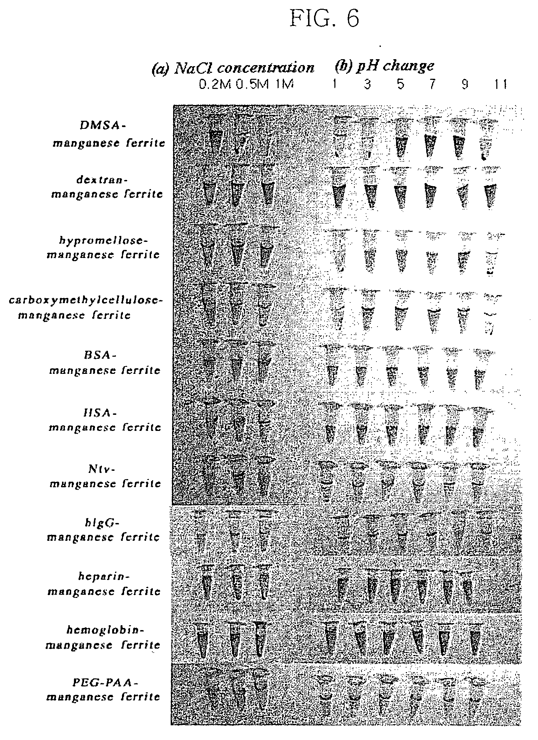

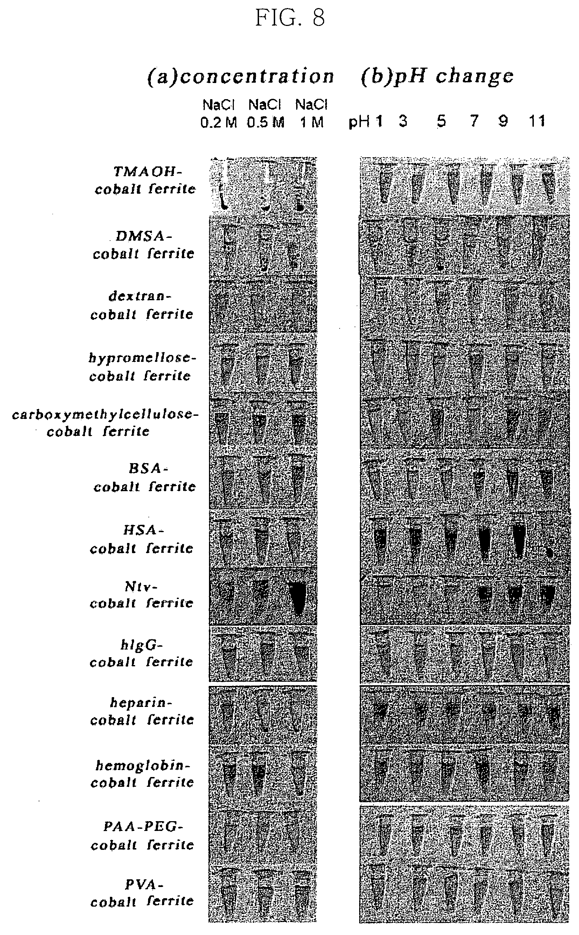

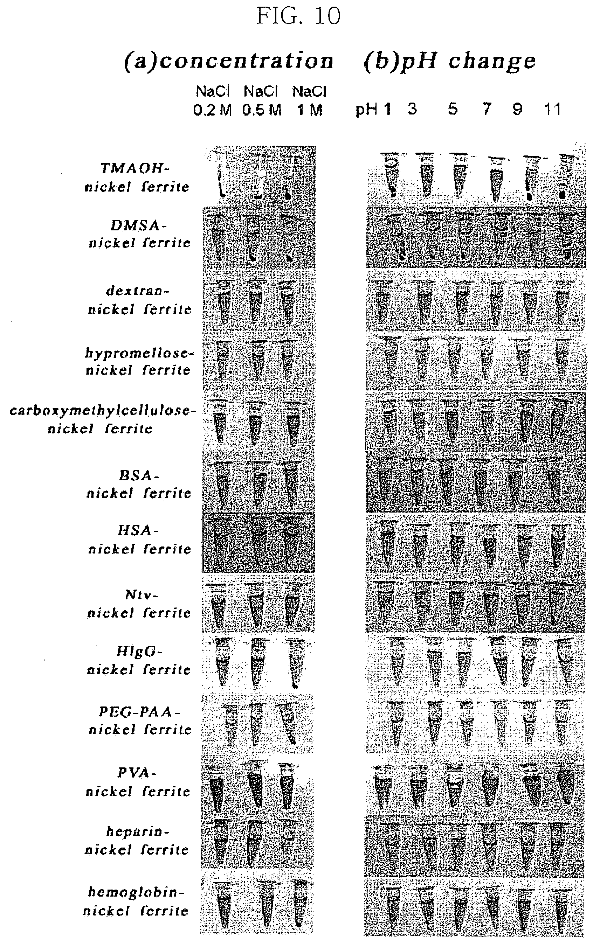

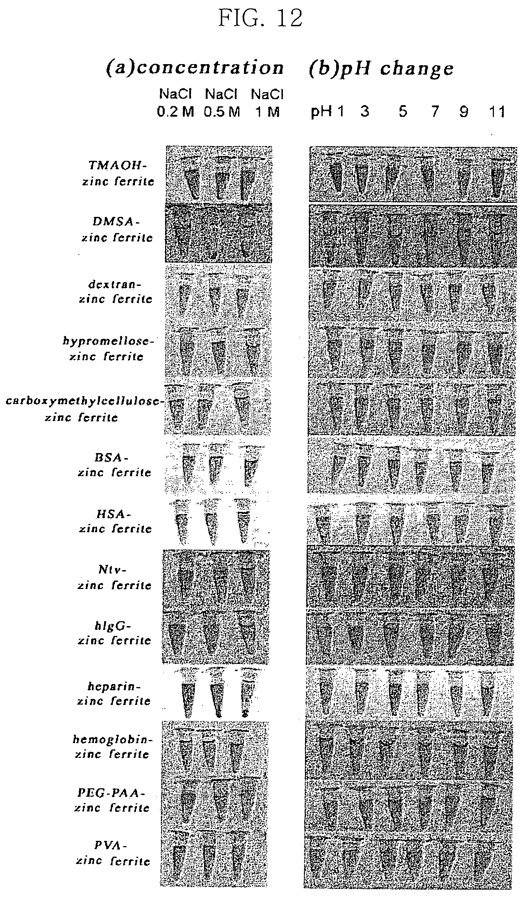

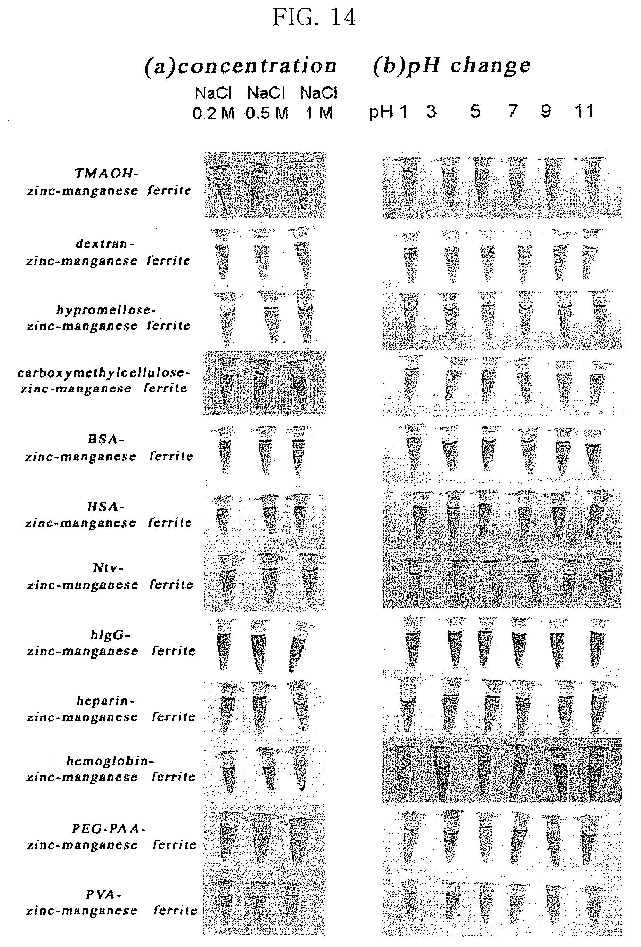

As shown in (a) of FIGS. 4, 6, 8, 10, 12, and 14, all of the water-soluble nanoparticles were stable at a NaCl concentration of 200 mM, and in particular, BSA-iron oxide, manganese ferrite, cobalt ferrite, nickel ferrite, zinc ferrite, and zinc-manganese ferrite nanoparticles; HSA-iron oxide, manganese ferrite, cobalt ferrite, nickel ferrite, zinc ferrite, and zinc-manganese ferrite nanoparticles; Ntv-iron oxide, manganese ferrite, cobalt ferrite, nickel ferrite, zinc ferrite, and zinc-manganese ferrite nanoparticles; hemoglobin-iron oxide, manganese ferrite, cobalt ferrite, nickel ferrite, zinc ferrite, and zinc-manganese ferrite nanoparticles; heparin-iron oxide, manganese ferrite, cobalt ferrite, nickel ferrite, zinc ferrite, and zinc-manganese ferrite nanoparticles; dextran-iron oxide, manganese ferrite, cobalt ferrite, nickel ferrite, zinc ferrite, and zinc-manganese ferrite nanoparticles; hypromellose-iron oxide, manganese ferrite, cobalt ferrite, nickel ferrite, zinc ferrite, and zinc-manganese ferrite nanoparticles; carboxymethylcellulose-iron oxide, manganese ferrite, cobalt ferrite, nickel ferrite, zinc ferrite, and zinc-manganese ferrite nanoparticles; PVA-iron oxide, manganese ferrite, cobalt ferrite, nickel ferrite, zinc ferrite, and zinc-manganese ferrite nanoparticles; and PAA-PEG-iron oxide, manganese ferrite, cobalt ferrite, nickel ferrite, zinc ferrite, and zinc-manganese ferrite nanoparticles were also stable at a concentration of 1 M or more. In contrast, the DMAP-Au and CTAB-Au nanoparticles, used as a control group representing the conventional method known in the prior art, were seen to be deposited at a NaCl concentration of 200 mM.

In addition, as shown in (b) of FIGS. 4, 6, 8, 10, 12, and 14, all of the water-soluble nanoparticles were stable at a pH of 7∼9, and in particular, BSA-iron oxide, manganese ferrite, cobalt ferrite, nickel ferrite, zinc ferrite, and zinc-manganese ferrite nanoparticles; HSA-iron oxide, manganese ferrite, cobalt ferrite, nickel ferrite, zinc ferrite, and zinc-manganese ferrite nanoparticles; Ntv-iron oxide, manganese ferrite, cobalt ferrite, nickel ferrite, zinc ferrite, and zinc-manganese ferrite nanoparticles; hemoglobin-iron oxide, manganese ferrite, cobalt ferrite, nickel ferrite, zinc ferrite, and zinc-manganese ferrite nanoparticles; heparin-iron oxide, manganese ferrite, cobalt ferrite, nickel ferrite, zinc ferrite, and zinc-manganese ferrite nanoparticles; dextran-iron oxide, manganese ferrite, cobalt ferrite, nickel ferrite, zinc ferrite, and zinc-manganese ferrite nanoparticles; carboxymethylcellulose-iron oxide, manganese ferrite, cobalt ferrite, nickel ferrite, zinc ferrite, and zinc-manganese ferrite nanoparticles; and PVA-iron oxide, manganese ferrite, cobalt ferrite, nickel ferrite, zinc ferrite, and zinc-manganese ferrite nanoparticles were also stable at a pH of 1 ∼11. However, the DMAP-Au was unstable at a pH of 1∼7 and was consequently precipitated, indicating low pH stability.

### Comparative Example 15: Synthesis of Water-Soluble Manganese Oxide Nanoparticles Coated with BSA (L_{I}-L_{III})

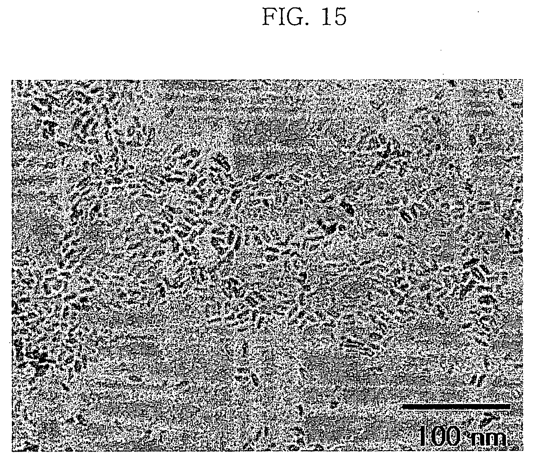

Water-insoluble manganese oxide nanoparticles were synthesized according to the technique disclosed in Korean Patent No. 604935, filed by the present inventors. All of the obtained nanoparticles were observed to have a thickness of 7 nm and a length of 15 nm, as intended, and to have a uniform size distribution and high crystallinity (FIG. 15).

The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). Further, 10 mg of BSA was dissolved in 1 ml of deionized water, and was then mixed with the precipitate, thus synthesizing manganese oxide nanoparticles coated with BSA. Subsequently, unreacted BSA was removed using a Sephacryl S-300 column, thereby yielding water-soluble manganese oxide nanoparticles coated with pure BSA.

### Comparative Example 16: Synthesis of Water-Soluble Manganese Oxide Nanoparticles Coated with HSA (L_{I}-L_{III})

As water-insoluble manganese oxide nanoparticles, the same manganese oxide nanoparticles as those of Example 15 were used. The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). Further, 10 mg of HSA was dissolved in 1 ml of deionized water, and was then mixed with the precipitate, thus synthesizing manganese oxide nanoparticles coated with HSA. Subsequently, unreacted HSA was removed using a Sephacryl S-300 column, thereby yielding water-soluble manganese oxide nanoparticles coated with pure HSA.

### Comparative Example 17: Synthesis of Water-Soluble Manganese Oxide Nanoparticles Coated with hIgG (L_{I}-L_{III})

As water-insoluble manganese oxide nanoparticles, the same manganese oxide nanoparticles as those of Example 15 were used. The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). Further, 10 mg of hIgG was dissolved in 1 ml of deionized water, and was then mixed with the precipitate, thus synthesizing manganese oxide nanoparticles coated with hIgG. Subsequently, unreacted hIgG was removed using a Sephacryl S-300 column, thereby yielding water-soluble manganese oxide nanoparticles coated with pure hIgG.

### Comparative Example 18: Synthesis of Water-Soluble Manganese Oxide Nanoparticles Coated with Ntv (L_{I}-L_{III})

As water-insoluble manganese oxide nanoparticles, the same manganese oxide nanoparticles as those of Example 15 were used. The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). Further, 10 mg of Ntv was dissolved in 1 ml of deionized water, and was then mixed with the precipitate, thus synthesizing manganese oxide nanoparticles coated with Ntv. Subsequently, unreacted Ntv was removed using a Sephacryl S-300 column, thereby yielding water-soluble manganese oxide nanoparticles coated with pure Ntv.

### Example 19: Synthesis of Water-Soluble Manganese Oxide Nanoparticles Coated with Dextran (L_{I}-L_{III})

As water-insoluble manganese oxide nanoparticles, the same manganese oxide nanoparticles as those of Example 15 were used. The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). Further, 10 mg of dextran, having a molecular weight of 10,000 Da, was dissolved in 1 ml of deionized water, and was then mixed with the precipitate, thus synthesizing manganese oxide nanoparticles coated with dextran. Subsequently, unreacted dextran was removed using a Sephacryl S-300 column, thereby yielding water-soluble manganese oxide nanoparticles coated with pure dextran.

### Example 20: Synthesis of Water-Soluble Manganese Oxide Nanoparticles Coated with Hypromellose (L_{I}-L_{III})

As water-insoluble manganese oxide nanoparticles, the same manganese oxide nanoparticles as those of Example 15 were used. The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). Further, 10 mg of hypromellose, having a molecular weight of 80,000 Da, was dissolved in 1 ml of deionized water, and was then mixed with the precipitate, thus synthesizing manganese oxide nanoparticles coated with hypromellose. Subsequently, unreacted hypromellose was removed using a Sephacryl S-300 column, thereby yielding water-soluble manganese oxide nanoparticles coated with pure hypromellose.

### Example 21: Synthesis of Water-Soluble Manganese Oxide Nanoparticles Coated with Carboxymethylcellulose (L_{I}-L_{III})

As water-insoluble manganese oxide nanoparticles, the same manganese oxide nanoparticles as those of Example 15 were used. The water-insoluble nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min). Further, 10 mg of carboxymethylcellulose, having a molecular weight of 90,000 Da, was dissolved in 1 ml of deionized water, and was then mixed with the precipitate, thus synthesizing manganese oxide nanoparticles coated with carboxymethylcellulose. Subsequently, unreacted carboxymethylcellulose was removed using a Sephacryl S-300 column, thereby yielding water-soluble manganese oxide nanoparticles coated with pure carboxymethylcellulose.

### Example 22: Synthesis of Water-Soluble Manganese Oxide Nanoparticles Coated with DMSA (L_{I}-L_{III}) without Crosslinking

As water-insoluble manganese oxide nanoparticles, the same manganese oxide nanoparticles as those of Example 15 were used. The water-insoluble nanoparticles (5 mg) were dissolved in 1 ml of toluene, after which the toluene solution was added with 0.5 ml of methanol in which 20 mg of 2,3-DMSA was dissolved. After about 24 hours, a blackish brown precipitate was formed, and this precipitate was separated through centrifugation at 2000 rpm at room temperature for 5 minutes and was then re-dispersed in 1 ml of deionized water, thus synthesizing manganese oxide nanoparticles coated with DMSA. Subsequently, unreacted DMSA was removed using a Sephadex G-25 column, thereby yielding water-soluble manganese oxide nanoparticles coated with pure DMSA.

### Example 23: Synthesis of Manganese Oxide Nanoparticles Coated with Tetramethylammonium Hydroxide (L_{I})

Water-insoluble manganese oxide nanoparticles were synthesized in the same manner as in Example 15. The obtained nanoparticles (5 mg) were dispersed in 1 ml of 1 M NMe₄OH butanol solution and were then mixed for about 5 minutes, until uniform. Thereafter, a blackish brown precipitate was formed, and this precipitate was separated through centrifugation (2000 rpm, room temperature, 5 min) and was then re-dispersed in 1 ml of deionized water, thereby yielding manganese oxide nanoparticles coated with tetramethylammonium hydroxide.

### Example 24: Analysis of Stability in Aqueous Solution of Water-Soluble Manganese Oxide Nanoparticles Coated with Phase Transfer Ligands

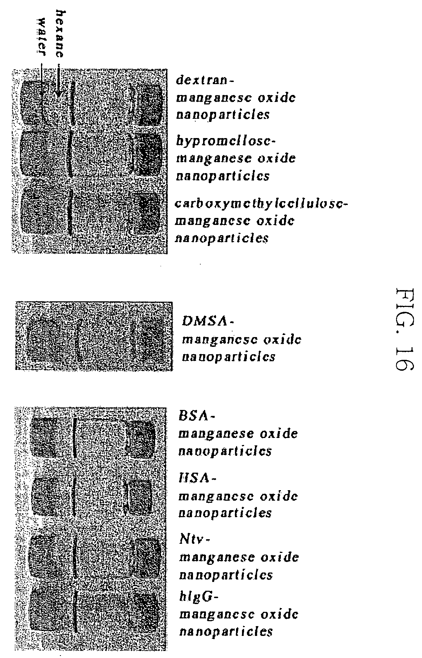

The solubility of the water-soluble nanoparticles synthesized in Examples 15 to 23 was analyzed through the same process as in Example 14. The results are shown in FIG. 16. The water-soluble manganese oxide nanoparticles were seen to be uniformly dispersed in the aqueous solution.

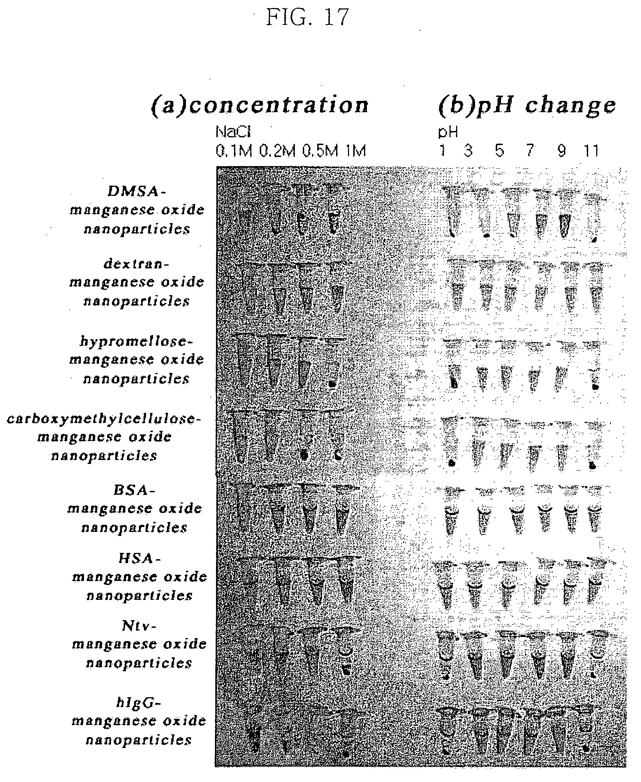

Further, the stability of the water-soluble nanoparticles synthesized in Examples 15 to 23, depending on changes in the salt concentration and pH of the aqueous solution, was analyzed through the same process as in Example 14. As is apparent from FIG. 17, the corresponding water-soluble nanoparticles were stable at a salt concentration of 1 M and a pH of 1∼11.

### Comparative Example 25: Synthesis of Water-Soluble Titanium Dioxide Nanoparticles Coated with BSA (L_{I}-L_{III})

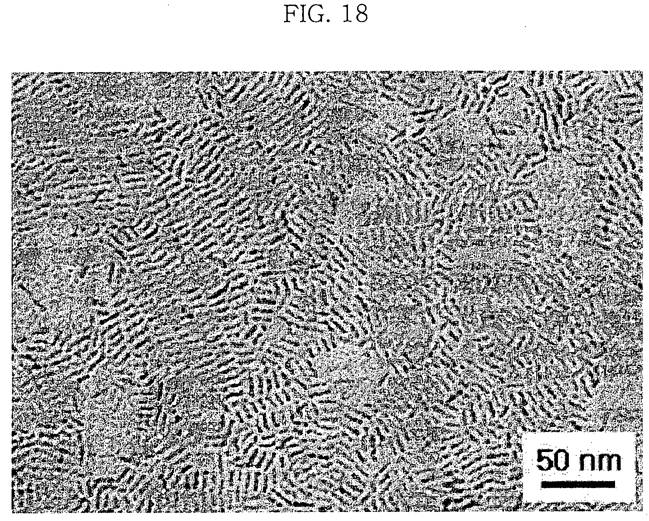

Water-insoluble titanium dioxide nanoparticles were synthesized according to the technique disclosed in Korean Patent No. 6049375, filed by the present inventors. All of the obtained nanoparticles were observed to have a thickness of 3 nm and a length of 20 nm, as intended, and to have a uniform size distribution and high crystallinity (FIG. 18).

The water-insoluble nanoparticles (5 mg) were dispersed in 1 ml of 1 M NMe₄OH butanol solution and were then mixed for about 5 minutes, until uniform. Thereafter, a white precipitate was formed, and this precipitate was separated through centrifugation (2000 rpm, room temperature, 5 min). Further, 10 mg of BSA was dissolved in 1 ml of deionized water, and was then mixed with the precipitate, thus synthesizing titanium dioxide nanoparticles coated with BSA. Subsequently, unreacted BSA was removed using a Sephacryl S-300 column, thereby yielding water-soluble titanium dioxide nanoparticles coated with pure BSA.

### Comparative Example 26: Synthesis of Water-Soluble Titanium Dioxide Nanoparticles Coated with HSA (L_{I}-L_{III})

As water-insoluble titanium dioxide nanoparticles, the same manganese oxide nanoparticles as those of Example 25 were used. The water-insoluble nanoparticles (5 mg) were dispersed in 1 ml of 1 M NMe₄OH butanol solution and were then mixed for about 5 minutes, until uniform. Thereafter, a white precipitate was formed, and this precipitate was separated through centrifugation (2000 rpm, room temperature, 5 min). Further, 10 mg of HSA was dissolved in 1 ml of deionized water, and was then mixed with the precipitate, thus synthesizing titanium dioxide nanoparticles coated with HSA. Subsequently, unreacted HSA was removed using a Sephacryl S-300 column, thereby yielding water-soluble titanium dioxide nanoparticles coated with pure HSA.

### Comparative Example 27: Synthesis of Water-Soluble Titanium Dioxide Nanoparticles Coated with hIgG (L_{I}-L_{III})

As water-insoluble titanium dioxide nanoparticles, the same titanium dioxide nanoparticles as those of Example 25 were used. The water-insoluble nanoparticles (5 mg) were dispersed in 1 ml of 1 M NMe₄OH butanol solution and were then mixed for about 5 minutes, until uniform. Thereafter, a white precipitate was formed, and this precipitate was separated through centrifugation (2000 rpm, room temperature, 5 min). Further, 10 mg of hIgG was dissolved in 1 ml of deionized water, and was then mixed with the precipitate, thus synthesizing titanium dioxide nanoparticles coated with hIgG. Subsequently, unreacted hIgG was removed using a Sephacryl S-300 column, thereby yielding water-soluble titanium dioxide nanoparticles coated with pure hIgG.

### Comparative Example 28: Synthesis of Water-Soluble Titanium Dioxide Nanoparticles Coated with Ntv (L_{I}-L_{III})

As water-insoluble titanium dioxide nanoparticles, the same titanium dioxide nanoparticles as those of Example 25 were used. The water-insoluble nanoparticles (5 mg) were dispersed in 1 ml of 1 M NMe₄OH butanol solution and were then mixed for about 5 minutes, until uniform. Thereafter, a white precipitate was formed, and this precipitate was separated through centrifugation (2000 rpm, room temperature, 5 min). Further, 10 mg of Ntv was dissolved in 1 ml of deionized water, and was then mixed with the precipitate, thus synthesizing titanium dioxide nanoparticles coated with Ntv. Subsequently, unreacted Ntv was removed using a Sephacryl S-300 column, thereby yielding water-soluble titanium dioxide nanoparticles coated with pure Ntv.

### Comparative Example 29: Synthesis of Water-Soluble Titanium Dioxide Nanoparticles Coated with Hemoglobin (L_{I}-L_{III})

As water-insoluble titanium dioxide nanoparticles, the same titanium dioxide nanoparticles as those of Example 25 were used. The water-insoluble nanoparticles (5 mg) were dispersed in 1 ml of 1 M NMe₄OH butanol solution and were then mixed for about 5 minutes, until uniform. Thereafter, a brown precipitate was formed, and this precipitate was separated through centrifugation (2000 rpm, room temperature, 5 min). Further, 10 mg of hemoglobin was dissolved in 1 ml of deionized water, and was then mixed with the precipitate, thus synthesizing titanium dioxide nanoparticles coated with hemoglobin. Subsequently, unreacted hemoglobin was removed using a Sephacryl S-300 column, thereby yielding water-soluble titanium dioxide nanoparticles coated with pure hemoglobin.

### Comparative Example 30: Synthesis of Titanium Dioxide Nanoparticles Coated with Heparin (L_{I}-L_{III})

As water-insoluble titanium dioxide nanoparticles, the same titanium dioxide nanoparticles as those of Example 25 were used. The water-insoluble nanoparticles (5 mg) were dispersed in 1 ml of 1 M NMe₄OH butanol solution and were then mixed for about 5 minutes, until uniform. Thereafter, a white precipitate was formed, and this precipitate was separated through centrifugation (2000 rpm, room temperature, 5 min). Further, 10 mg of heparin was dissolved in 1 ml of deionized water, and was then mixed with the precipitate, thus synthesizing titanium dioxide nanoparticles coated with heparin. Subsequently, unreacted heparin was removed using a Sephacryl S-300 column, thereby yielding water-soluble titanium dioxide nanoparticles coated with pure heparin.

### Example 31: Synthesis of Water-Soluble Titanium Dioxide Nanoparticles Coated with Dextran (L_{I}-L_{III})

As water-insoluble titanium dioxide nanoparticles, the same titanium dioxide nanoparticles as those of Example 25 were used. The water-insoluble nanoparticles (5 mg) were dispersed in 1 ml of 1 M NMe₄OH butanol solution and were then mixed for about 5 minutes, until uniform. Thereafter, a white precipitate was formed, and this precipitate was separated through centrifugation (2000 rpm, room temperature, 5 min). Further, 10 mg of dextran, having a molecular weight of 10,000 Da, was dissolved in 1 ml of deionized water, and was then mixed with the precipitate, thus synthesizing titanium dioxide nanoparticles coated with dextran. Subsequently, unreacted dextran was removed using a Sephacryl S-300 column, thereby yielding water-soluble titanium dioxide nanoparticles coated with pure dextran.

### Example 32: Synthesis of Water-Soluble Titanium Dioxide Nanoparticles Coated with Hypromellose (L_{I}-L_{III})

As water-insoluble titanium dioxide nanoparticles, the same titanium dioxide nanoparticles as those of Example 25 were used. The water-insoluble nanoparticles (5 mg) were dispersed in 1 ml of 1 M NMe₄OH butanol solution and were then mixed for about 5 minutes, until uniform. Thereafter, a white precipitate was formed, and this precipitate was separated through centrifugation (2000 rpm, room temperature, 5 min). Further, 10 mg of hypromellose, having a molecular weight of 80,000 Da, was dissolved in 1 ml of deionized water, and was then mixed with the precipitate, thus synthesizing titanium dioxide nanoparticles coated with hypromellose. Subsequently, unreacted hypromellose was removed using a Sephacryl S-300 column, thereby yielding water-soluble titanium dioxide nanoparticles coated with pure hypromellose.

### Example 33: Synthesis of Water-Soluble Titanium Dioxide Nanoparticles Coated with Carboxymethylcellulose (L_{I}-L_{III})

As water-insoluble titanium dioxide nanoparticles, the same titanium dioxide nanoparticles as those of Example 25 were used. The water-insoluble nanoparticles (5 mg) were dispersed in 1 ml of 1 M NMe₄OH butanol solution and were then mixed for about 5 minutes, until uniform. Thereafter, a white precipitate was formed, and this precipitate was separated through centrifugation (2000 rpm, room temperature, 5 min). Further, 10 mg of carboxymethylcellulose, having a molecular weight of 90,000 Da, was dissolved in 1 ml of deionized water, and was then mixed with the precipitate, thus synthesizing titanium dioxide nanoparticles coated with carboxymethylcellulose. Subsequently, unreacted carboxymethylcellulose was removed using a Sephacryl S-300 column, thereby yielding water-soluble titanium dioxide nanoparticles coated with pure carboxymethylcellulose.

### Example 34: Synthesis of Water-Soluble Titanium Dioxide Nanoparticles Coated with PVA (L_{I})

As water-insoluble titanium dioxide nanoparticles, the same titanium dioxide nanoparticles as those of Example 25 were used. The water-insoluble nanoparticles (5 mg) were dispersed in 1 ml of 1 M NMe₄OH butanol solution and were then mixed for about 5 minutes, until uniform. Thereafter, a white precipitate was formed, and this precipitate was separated through centrifugation (2000 rpm, room temperature, 5 min). Further, 10 mg of PVA, having a molecular weight of 10,000 Da, was dissolved in 1 ml of deionized water, and was then mixed with the precipitate, thus synthesizing titanium dioxide nanoparticles coated with PVA. Subsequently, unreacted PVA was removed using a Sephacryl G-25 column, thereby yielding water-soluble titanium dioxide nanoparticles coated with pure PVA.

### Example 35: Synthesis of Water-Soluble Titanium Dioxide Nanoparticles Coated with PAA-PEG (L_{I}-L_{II})

PAA-PEG was prepared in the same manner as in Example 11. As water-insoluble titanium dioxide nanoparticles, the same titanium dioxide nanoparticles as those of Example 25 were used. The water-insoluble nanoparticles (5 mg) were dispersed in a solution (5 mg/ml) of PAA-PEG dissolved in 1 ml of ethanol, and were then mixed for about 10 hours, until uniform. Thereafter, a white precipitate was formed, and this precipitate was separated through centrifugation (2000 rpm, room temperature, 5 min). Further, the precipitate was dissolved in 1 ml of deionized water, thus synthesizing titanium dioxide nanoparticles coated with PAA-PEG. Subsequently, unreacted PAA-PEG was removed using a Sephacryl G-25 column, thereby yielding water-soluble titanium dioxide nanoparticles coated with pure PAA-PEG.

### Example 36: Synthesis of Water-Soluble Titanium Dioxide Nanoparticles Coated with DMSA (L_{I}-L_{III}) without Crosslinking

As water-insoluble titanium dioxide nanoparticles, the same titanium dioxide nanoparticles as those of Example 25 were used. The water-insoluble nanoparticles (5 mg) were dissolved in 1 ml of toluene, after which the toluene solution was added with 0.5 ml of methanol in which 20 mg of 2,3-DMSA was dissolved. After about 24 hours, a white precipitate was formed, and this precipitate was separated through centrifugation at 2000 rpm at room temperature for 5 min and was then re-dispersed in 1 ml of deionized water, thus synthesizing titanium dioxide nanoparticles coated with DMSA. Subsequently, unreacted DMSA was removed using a Sephadex G-25 column, thereby yielding water-soluble titanium dioxide nanoparticles coated with pure DMSA.

### Example 37: Synthesis of Titanium Dioxide Nanoparticles Coated with Tetramethylammonium Hydroxide (L_{I})

Water-insoluble titanium dioxide nanoparticles were synthesized in the same manner as in Example 25. The obtained nanoparticles (5 mg) were dispersed in 1 ml of 1 M NMe₄OH butanol solution and were then mixed for about 5 minutes, until uniform. Thereafter, a white precipitate was formed, and this precipitate was separated through centrifugation (2000 rpm, room temperature, 5 min) and was then re-dispersed in 1 ml of deionized water, thereby yielding titanium dioxide nanoparticles coated with tetramethylammonium hydroxide.

### Example 38: Analysis of Stability in Aqueous Solution of Water-Soluble Titanium Dioxide Nanoparticles Coated with Phase Transfer Ligands

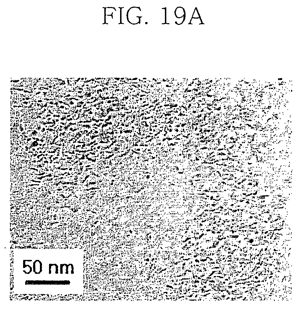

20 µl of a solution containing the water-soluble titanium dioxide nanoparticles synthesized in Example 25 was dropped on a TEM grid (Ted Pella inc.) coated with a carbon film, dried for about 30 min, and then observed using an electron microscope (EF-TEM, Zeiss, acceleration voltage 100 kV). As is apparent from FIG. 19A, the synthesized water-soluble titanium dioxide nanoparticles were seen to have a uniform size and to be uniformly dispersed.

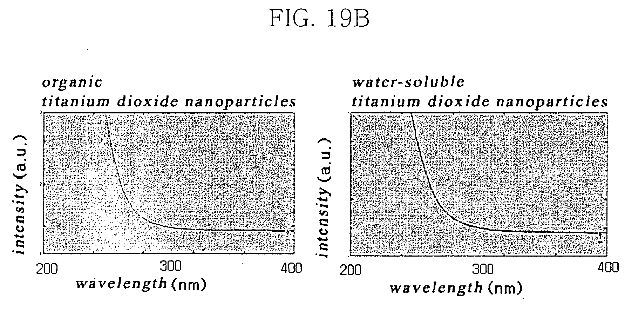

Further, through analysis of the UV-Vis absorption spectrum, whether the water-soluble titanium dioxide nanoparticles were contained in the aqueous solution was determined. As shown in FIG. 19B, the water-soluble nanoparticles had an absorption spectrum that was almost the same as that of organic titanium oxide. Thereby, it could be seen that the water-soluble titanium dioxide nanoparticles were dissolved in the aqueous solution.

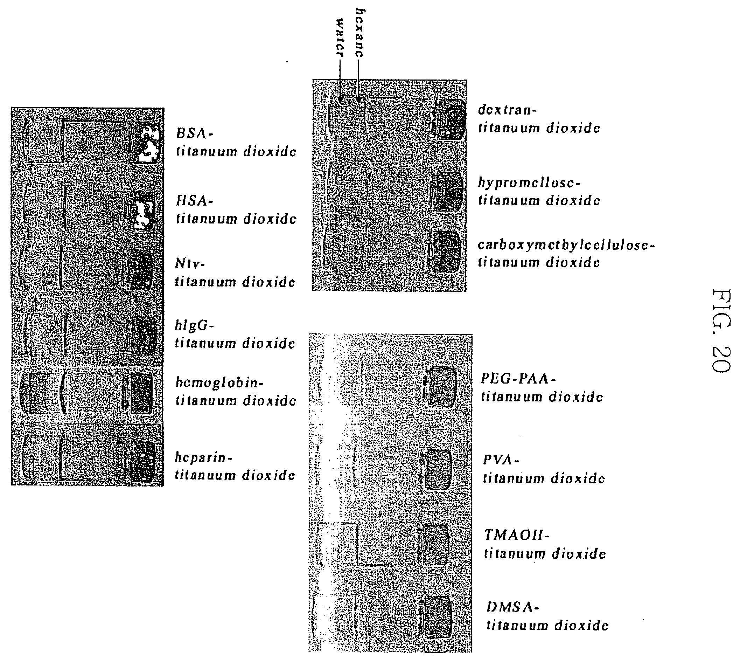

The solubility of the water-soluble titanium dioxide nanoparticles synthesized in Examples 25 to 37 was analyzed through the same process as in Example 14. The results are shown in FIG. 20. The water-soluble titanium dioxide nanoparticles could be seen to be uniformly dispersed in the aqueous solution.

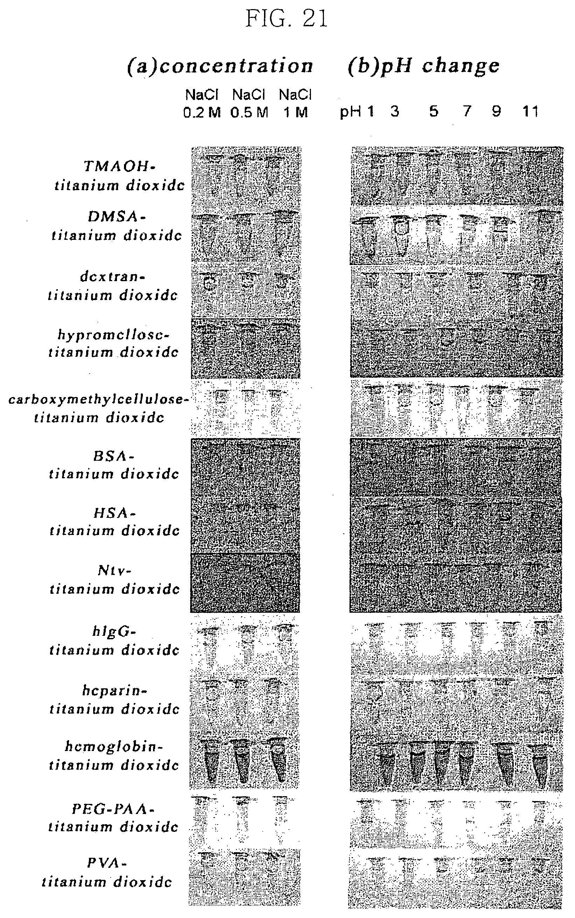

The stability of the water-soluble nanoparticles synthesized in Examples 25 to 37, depending on changes in the salt concentration and pH of the aqueous solution, was analyzed through the same process as in Example 14. As shown in FIG. 21, the corresponding water-soluble nanoparticles were stable at a salt concentration of 1 M and a pH of 1∼11.

### Comparative Example 39: Synthesis of Water-Soluble FePt Nanoparticles Coated with BSA (L_{I}-L_{III})

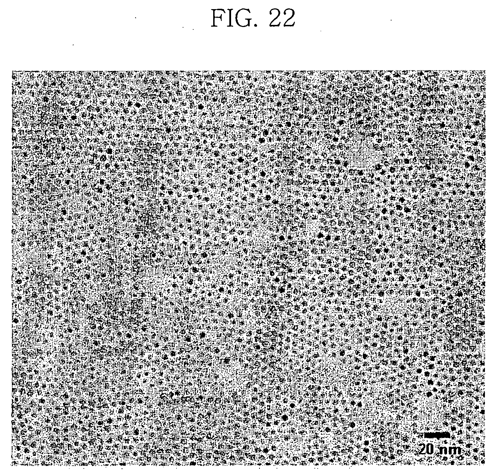

FePt nanoparticles having a particle size of 6 nm were synthesized using a method known in the art (Journal of the American Chemical Society, 2004, vol.126, p.8394). All of the obtained nanoparticles were observed to have a particle size of 6 nm, as intended, and to have a uniform size distribution and high crystallinity (FIG. 22).

The water-insoluble nanoparticles (5 mg) were dispersed in 1 ml of 1 M NMe₄OH butanol solution and were then mixed for about 5 minutes, until uniform. Thereafter, a black precipitate was formed, and this precipitate was separated through centrifugation (2000 rpm, room temperature, 5 min). Further, 10 mg of BSA was dissolved in 1 ml of deionized water, and was then mixed with the precipitate, thus synthesizing FePt nanoparticles coated with BSA. Subsequently, unreacted BSA was removed using a Sephacryl S-300 column, thereby yielding water-soluble FePt nanoparticles coated with pure BSA.

### Comparative Example 40: Synthesis of Water-Soluble FePt Nanoparticles Coated with HSA (L_{I}-L_{III})

As water-insoluble FePt nanoparticles, the same FePt nanoparticles as those of Example 39 were used. The water-insoluble nanoparticles (5 mg) were dispersed in 1 ml of 1 M NMe₄OH butanol solution and were then mixed for about 5 minutes, until uniform. Thereafter, a black precipitate was formed, and this precipitate was separated through centrifugation (2000 rpm, room temperature, 5 min). Further, 10 mg of HSA was dissolved in 1 ml of deionized water, and was then mixed with the precipitate, thus synthesizing FePt nanoparticles coated with HSA. Subsequently, unreacted HSA was removed using a Sephacryl S-300 column, thereby yielding water-soluble FePt nanoparticles coated with pure HSA.

### Comparative Example 41: Synthesis of Water-Soluble FePt Nanoparticles Coated with hIgG (L_{I}-L_{III})

As water-insoluble FePt nanoparticles, the same FePt nanoparticles as those of Example 39 were used. The water-insoluble nanoparticles (5 mg) were dispersed in 1 ml of 1 M NMe₄OH butanol solution and were then mixed for about 5 minutes, until uniform. Thereafter, a black precipitate was formed, and this precipitate was separated through centrifugation (2000 rpm, room temperature, 5 min). Further, 10 mg of hIgG was dissolved in 1 ml of deionized water, and was then mixed with the precipitate, thus synthesizing FePt nanoparticles coated with hIgG. Subsequently, unreacted hIgG was removed using a Sephacryl S-300 column, thereby yielding water-soluble FePt nanoparticles coated with pure hIgG.

### Comparative Example 42: Synthesis of Water-Soluble FePt Nanoparticles Coated with Ntv (L_{I}-L_{III})

As water-insoluble FePt nanoparticles, the same FePt nanoparticles as those of Example 39 were used. The water-insoluble nanoparticles (5 mg) were dispersed in 1 ml of 1 M NMe₄OH butanol solution and were then mixed for about 5 minutes, until uniform. Thereafter, a black precipitate was formed, and this precipitate was separated through centrifugation (2000 rpm, room temperature, 5 min). Further, 10 mg of Ntv was dissolved in 1 ml of deionized water, and was then mixed with the precipitate, thus synthesizing FePt nanoparticles coated with Ntv. Subsequently, unreacted Ntv was removed using a Sephacryl S-300 column, thereby yielding water-soluble FePt nanoparticles coated with pure Ntv.

### Comparative Example 43: Synthesis of Water-Soluble FePt Nanoparticles Coated with Hemoglobin (L_{I}-L_{III})

As water-insoluble FePt nanoparticles, the same FePt nanoparticles as those of Example 39 were used. The water-insoluble nanoparticles (5 mg) were dispersed in 1 ml of 1 M NMe₄OH butanol solution and were then mixed for about 5 minutes, until uniform. Thereafter, a black precipitate was formed, and this precipitate was separated through centrifugation (2000 rpm, room temperature, 5 min). Further, 10 mg of hemoglobin was dissolved in 1 ml of deionized water, and was then mixed with the precipitate, thus synthesizing FePt nanoparticles coated with hemoglobin. Subsequently, unreacted hemoglobin was removed using a Sephacryl S-300 column, thereby yielding water-soluble FePt nanoparticles coated with pure hemoglobin.

### Comparative Example 44: Synthesis of Water-Soluble FePt Nanoparticles Coated with Heparin (L_{I}-L_{III})

As water-insoluble FePt nanoparticles, the same FePt nanoparticles as those of Example 39 were used. The water-insoluble nanoparticles (5 mg) were dispersed in 1 ml of 1 M NMe₄OH butanol solution and were then mixed for about 5 minutes, until uniform. Thereafter, a black precipitate was formed, and this precipitate was separated through centrifugation (2000 rpm, room temperature, 5 min). Further, 10 mg of heparin was dissolved in 1 ml of deionized water, and was then mixed with the precipitate, thus synthesizing FePt nanoparticles coated with heparin. Subsequently, unreacted heparin was removed using a Sephacryl S-300 column, thereby yielding water-soluble FePt nanoparticles coated with pure heparin.

### Comparative Example 45: Synthesis of Water-Soluble FePt Nanoparticles Coated with Dextran (L_{I}-L_{III})

As water-insoluble FePt nanoparticles, the same FePt nanoparticles as those of Example 39 were used. The water-insoluble nanoparticles (5 mg) were dispersed in 1 ml of 1 M NMe₄OH butanol solution and were then mixed for about 5 minutes, until uniform. Thereafter, a black precipitate was formed, and this precipitate was separated through centrifugation (2000 rpm, room temperature, 5 min). Further, 10 mg of dextran, having a molecular weight of 10,000 Da, was dissolved in 1 ml of deionized water, and was then mixed with the precipitate, thus synthesizing FePt nanoparticles coated with dextran. Subsequently, unreacted dextran was removed using a Sephacryl S-300 column, thereby yielding water-soluble FePt nanoparticles coated with pure dextran.

### Comparative Example 46: Synthesis of Water-Soluble FePt Nanoparticles Coated with Hypromellose (L_{I}-L_{III})

As water-insoluble FePt nanoparticles, the same FePt nanoparticles as those of Example 39 were used. The water-insoluble nanoparticles (5 mg) were dispersed in 1 ml of 1 M NMe₄OH butanol solution and were then mixed for about 5 minutes, until uniform. Thereafter, a black precipitate was formed, and this precipitate was separated through centrifugation (2000 rpm, room temperature, 5 min). Further, 10 mg of hypromellose, having a molecular weight of 80,000 Da, was dissolved in 1 ml of deionized water, and was then mixed with the precipitate, thus synthesizing FePt nanoparticles coated with hypromellose. Subsequently, unreacted hypromellose was removed using a Sephacryl S-300 column, thereby yielding water-soluble FePt nanoparticles coated with pure hypromellose.

### Comparative Example 47: Synthesis of Water-Soluble FePt Nanoparticles Coated with Carboxymethylcellulose (L_{I}-L_{III})

As water-insoluble FePt nanoparticles, the same FePt nanoparticles as those of Example 39 were used. The water-insoluble nanoparticles (5 mg) were dispersed in 1 ml of 1 M NMe₄OH butanol solution and were then mixed for about 5 minutes, until uniform. Thereafter, a black precipitate was formed, and this precipitate was separated through centrifugation (2000 rpm, room temperature, 5 min). Further, 10 mg of carboxymethylcellulose, having a molecular weight of 90,000 Da, was dissolved in 1 ml of deionized water, and was then mixed with the precipitate, thus synthesizing FePt nanoparticles coated with carboxymethylcellulose. Subsequently, unreacted carboxymethylcellulose was removed using a Sephacryl S-300 column, thereby yielding water-soluble FePt nanoparticles coated with pure carboxymethylcellulose.

### Comparative Example 48: Synthesis of Water-Soluble FePt Nanoparticles Coated with PVA (L_{I})

As water-insoluble FePt nanoparticles, the same FePt nanoparticles as those of Example 39 were used. The water-insoluble nanoparticles (5 mg) were dispersed in 1 ml of 1 M NMe₄OH butanol solution, and were then mixed for about 5 minutes, until uniform. Thereafter, a black precipitate was formed, and this precipitate was separated through centrifugation (2000 rpm, room temperature, 5 min). Further, 10 mg of PVA, having a molecular weight of 10,000 Da, was dissolved in 1 ml of deionized water, and was then mixed with the precipitate, thus synthesizing FePt nanoparticles coated with PVA. Subsequently, unreacted PVA was removed using a Sephacryl G-25 column, thereby yielding water-soluble FePt nanoparticles coated with pure PVA.

### Comparative Example 49: Synthesis of Water-Soluble FePt Nanoparticles Coated with PAA-PEG (L_{I}-L_{II})

PAA-PEG was prepared in the same manner as in Example 11.

As water-insoluble FePt nanoparticles, the same FePt nanoparticles as those of Example 39 were used. The water-insoluble nanoparticles (5 mg) were dispersed in a solution (5 mg/ml) of PAA-PEG dissolved in 1 ml of ethanol, and were then mixed for about 10 hours, until uniform. Thereafter, a black precipitate was formed, and this precipitate was separated through centrifugation (2000 rpm, room temperature, 5 min). Further, the precipitate was dissolved in 1 ml of deionized water, thus synthesizing FePt nanoparticles coated with PAA-PEG. Subsequently, unreacted PAA-PEG was removed using a Sephacryl G-25 column, thereby yielding water-soluble FePt nanoparticles coated with pure PAA-PEG.

### Comparative Example 50: Synthesis of Water-Soluble FePt Nanoparticles Coated with DMSA (L_{I}-L_{III}) without Crosslinking

As water-insoluble FePt nanoparticles, the same FePt nanoparticles as those of Example 39 were used. The water-insoluble nanoparticles (5 mg) were dissolved in 1 ml of toluene, after which the toluene solution was added with 0.5 ml of methanol in which 20 mg of 2,3-DMSA was dissolved. After about 24 hours, a black precipitate was formed, and this precipitate was separated through centrifugation at 2000 rpm at room temperature for 5 minutes, and was then re-dispersed in 1 ml of deionized water, thus synthesizing FePt nanoparticles coated with DMSA. Subsequently, unreacted DMSA was removed using a Sephadex G-25 column, thereby yielding water-soluble FePt nanoparticles coated with pure DMSA.

### Comparative Example 51: Synthesis of FePt Nanoparticles Coated with Tetramethylammonium Hydroxide (L_{I})

Water-insoluble FePt nanoparticles were synthesized in the same manner as in Example 43. The obtained nanoparticles (5 mg) were dispersed in 1 ml of 1 M NMe₄OH butanol solution and were then mixed for about 5 minutes, until uniform. Thereafter, a black precipitate was formed, and this precipitate was separated through centrifugation (2000 rpm, room temperature, 5 min), and was then re-dispersed in 1 ml of deionized water, thereby yielding FePt nanoparticles coated with tetramethylammonium hydroxide.

### Comparative Example 52: Analysis of Stability in Aqueous Solution of Water-Soluble FePt Nanoparticles Coated with Phase Transfer Ligands

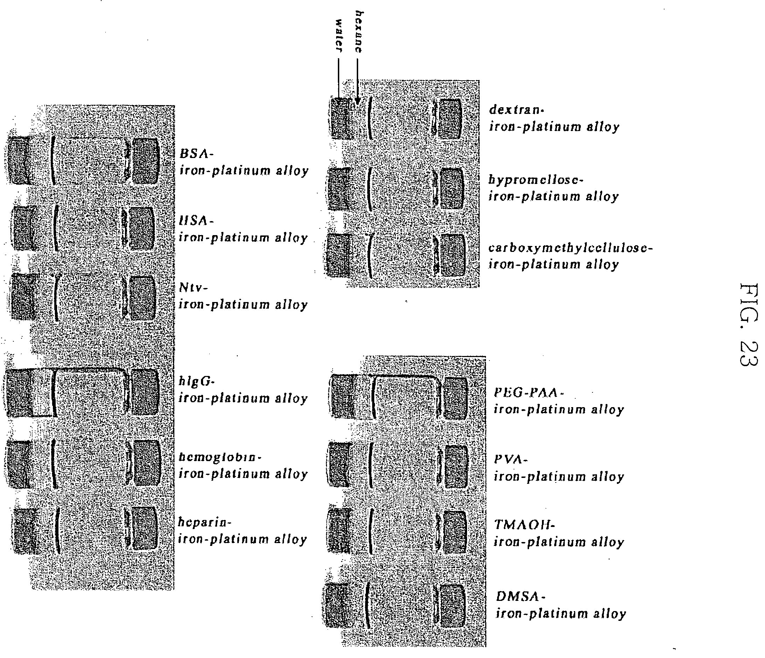

The solubility of the water-soluble nanoparticles synthesized in Examples 39 to 51 was analyzed through the same process as in Example 14. The results are shown in FIG. 23. The water-soluble FePt nanoparticles could be seen to be uniformly dispersed in the aqueous solution.

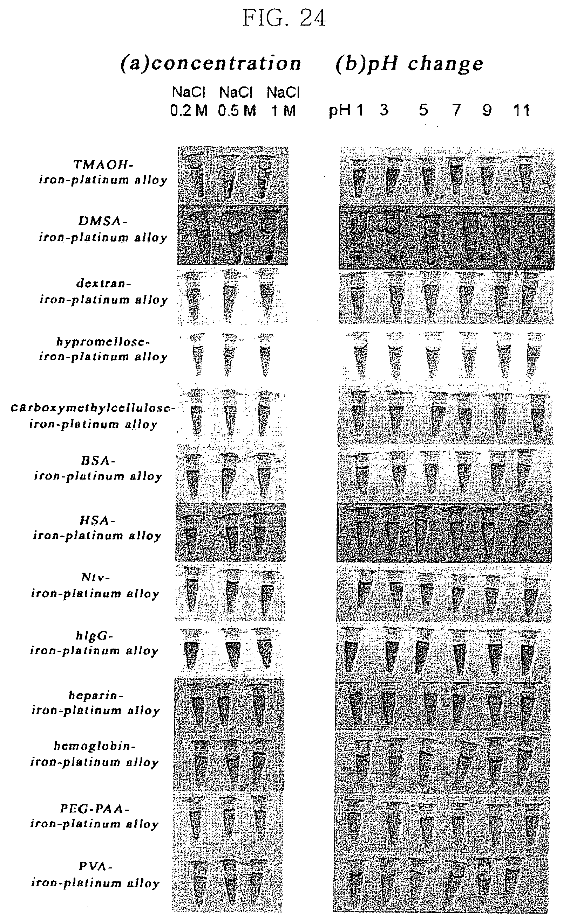

The stability of the water-soluble nanoparticles synthesized in Examples 39 to 51, depending on changes in the salt concentration and pH of the aqueous solution, was analyzed through the same process as in Example 14. As shown in FIG. 24, the corresponding water-soluble nanoparticles were stable at a salt concentration of 1 M and a pH of 1∼11.

### Comparative Example 53: Synthesis of Water-Soluble Multicomponent Hybrid Structure (FePt-Au) Nanoparticles

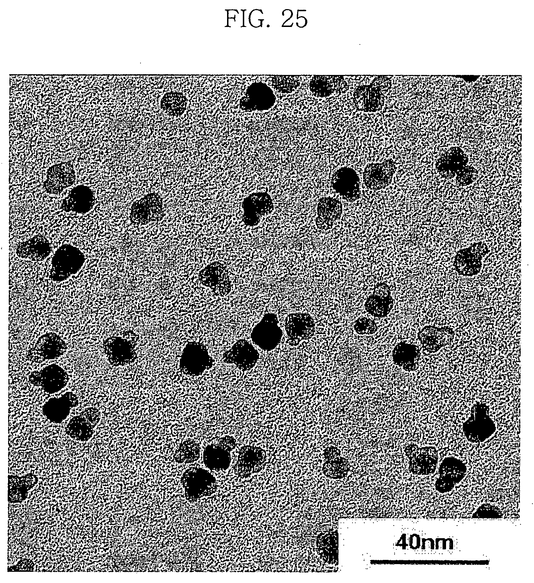

FePt nanoparticles having a particle size of 6 nm were synthesized using a method known in the art (Journal of the American Chemical Society, 2004, vol.126, p.8394). After FePt (5 mg), AuClPPh₃ (0.24 mg), and hexadecylamine (46 mg) were dissolved in 1,2-dichlorobenzene (5 ml), the solution was heated to 70°C, and was then subjected to bubbling using H₂(4%)/N₂ for 30 min. The solution was cooled to room temperature, added with ethanol (7 ml), and then centrifuged, thus obtaining FePt-Au nanoparticle powder as a precipitate. It could be seen that the water-insoluble nanoparticles thus obtained had a particle size of about 6 nm (FePt)-8 nm (Au) and also had a uniform size distribution (FIG. 25).

The water-insoluble FePt-Au nanoparticles (5 mg) were mixed with dihydrofolic acid-polyethyleneglycol (100 µl, MW: 600 Da) dissolved in ethanol (400 µl), and then the mixture was allowed to react for 3 hours. After the completion of the reaction, the resultant nanoparticles were added with distilled water (1 ml), and then unreacted dihydrofolic acid-polyethyleneglycol was removed using a Sephadex G-25 column, thus preparing pure water-soluble multicomponent hybrid structure nanoparticles.

### Comparative Example 54: Analysis of Stability in Aqueous Solution of Water-Soluble Multicomponent Hybrid Structure (FePt-Au) Nanoparticles

The stability in aqueous solution of the water-soluble core-shell nanoparticles prepared in Example 53 was evaluated according to the same process as in Example 14.

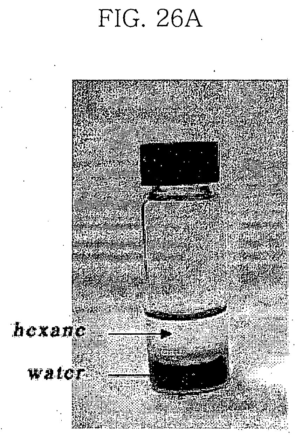

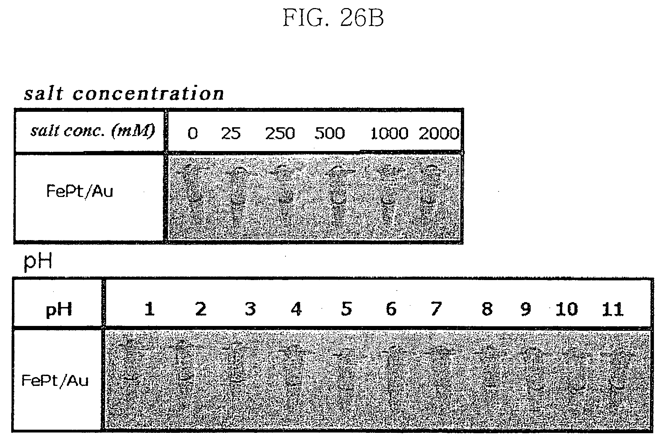

As shown in FIGS. 26A and 26B, all of the water-soluble multicomponent hybrid structure (FePt-Au) nanoparticles were stable in the aqueous solution, and furthermore, were stable in a 2 M NaCl solution and at a pH of 1∼12.

### Comparative Example 55: Synthesis of Water-Soluble Multicomponent Hybrid Structure (Co@Pt-Au) Nanoparticles

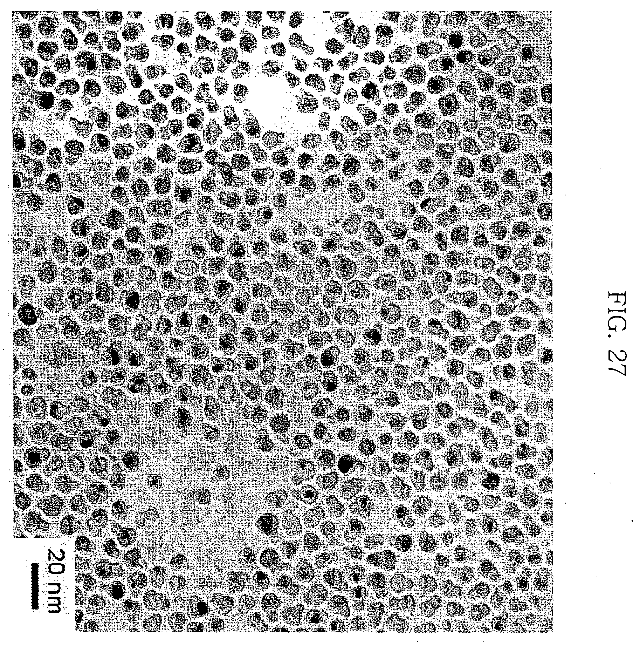

Core-shell Co@Pt nanoparticles having a particle size of 6 nm were synthesized using a method known in the art (Journal of the American Chemical Society, 2001, vol.123, p.5743). After Co@Pt (5 mg), AuClPPh₃ (0.24 mg), and hexadecylamine (46 mg) were dissolved in 1,2-dichlorobenzene (5 ml), the solution was heated to 70°C and was then subjected to bubbling using H₂(4%)/N₂ for 30 min. The solution was cooled to room temperature, added with ethanol (7 ml), and then centrifuged, thus obtaining Co@Pt-Au nanoparticle powder as a precipitate. It could be seen that the water-insoluble nanoparticles thus obtained had a particle size of about 6 nm (Co@Pt)-8 nm (Au) and also had a uniform size distribution (FIG. 27).

The water-insoluble Co@Pt-Au nanoparticles (5 mg) were mixed with dihydrofolic acid-polyethyleneglycol (100 µl, MW: 600) dissolved in ethanol (400 µl) and then the mixture was allowed to react for 3 hours. After the completion of the reaction, the resultant nanoparticles were added with distilled water (1 ml), after which unreacted dihydrofolic acid-polyethyleneglycol was removed using a Sephadex G-25 column, thus preparing pure water-soluble multicomponent hybrid structure Co@Pt-Au nanoparticles.

### Comparative Example 56: Analysis of Stability in Aqueous Solution of Water-Soluble Multicomponent Hybrid Structure (Co@Pt-Au) Nanoparticles

The stability in aqueous solution of the water-soluble core-shell nanoparticles prepared in Example 55 was evaluated according to the same process as in Example 14.

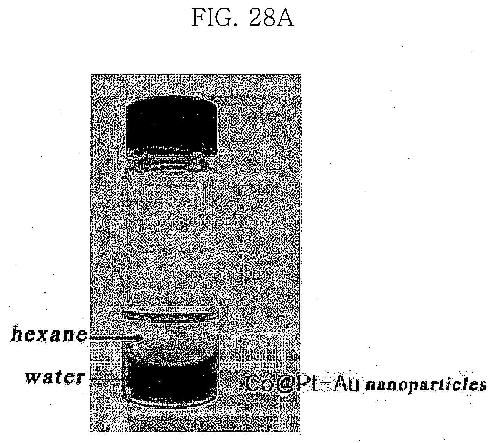

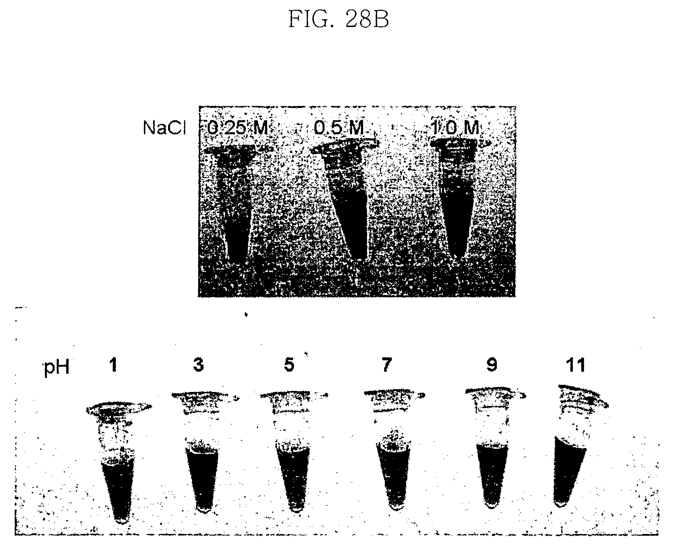

As shown in FIGS. 28A and 28B, all of the water-soluble multicomponent hybrid structure (Co@Pt-Au) nanoparticles were stable in the aqueous solution, and were also stable in a 2 M NaCl solution and at a pH of 1∼12.

### Comparative Example 57: Synthesis of BSA-Iron Oxide Nanoparticles having Ntv as Active Component

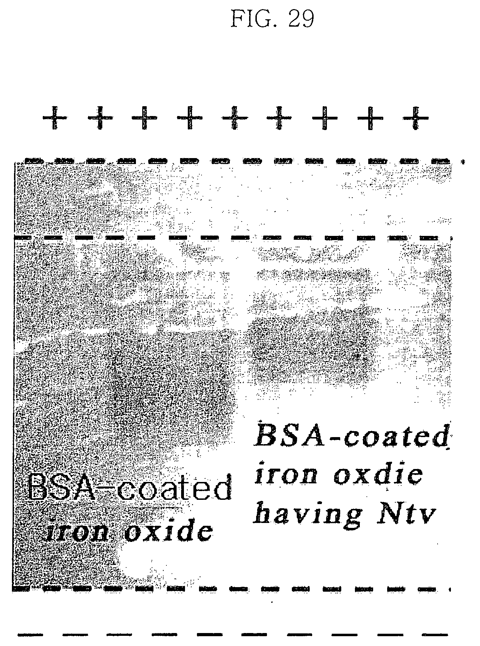

0.2 mg of BSA-Fe₃O₄ and 0.2 mg of Ntv were dissolved in 100 µl of 10 mM PBS (pH 7.2), after which EDC (N-(3-dimethylaminopropyl)-N'-ethylcarbodiimide hydrochloride, Pierce) and NHS (N-hydroxysulfosuccinimide, Pierce) were added to realize concentrations of 5 mM EDC and 2 mM NHS, respectively. The solution was allowed to react at room temperature for 3 hours, and then the reaction product was separated using a Sephacryl S-300 column, thus obtaining pure BSA-iron oxide nanoparticles having Ntv (FIG. 29).

### Comparative Example 58: Effect of MRI Signal Amplification of Water-Soluble Magnetic Nanoparticles coated with Phase Transfer Ligand

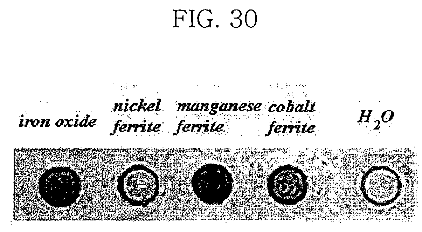

The iron oxide, manganese ferrite, cobalt ferrite, and nickel ferrite nanoparticles, prepared in Example 1, were diluted to a concentration of 100 mM, after which the signal amplification effect thereof was observed using SENSE™ Flex-M (14 cm x 17 cm flexible coil) in a 3T MRI (Achieva: Philips Medical Systems, Best, The Netherlands) system. Specific parameters were as follows: resolution = 156 x 156 mm, segment thickness = 0.6 mm, TR = 3000 ms, TE = 100 ms, and image excitation number = 1. Compared to the signals of water, all of the prepared water-soluble magnetic nanoparticles were seen to have amplified signals (FIG. 30).

### Comparative Example 59: Material Detection of Water-Soluble Iron Oxide Nanoparticles Coated with Phase Transfer Ligand using MRI

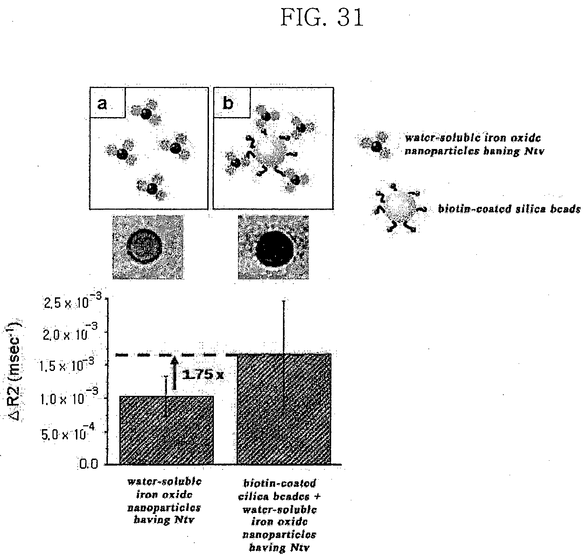

The aqueous solution (100 mM) containing the water-soluble iron oxide nanoparticles having Ntv, prepared in Example 55, was mixed with 100 mg of biotin-coated silica nanoparticles (100 nm, Microspheres-nanospheres, USA), the mixture was allowed to react for 30 min, and then the signals were measured using the same 3T MRI system as in Example 58. The MRI signals of the water-soluble iron oxide nanoparticles mixed with the biotin-coated silica could be seen to be amplified 1.7 times as much as the MRI signals of the iron oxide nanoparticles at the same concentration (FIG. 31).

### Comparative Example 60: Effect of Material Separation of Water-Soluble Iron Oxide Nanoparticles Coated with Phase Transfer Ligand using Magnet

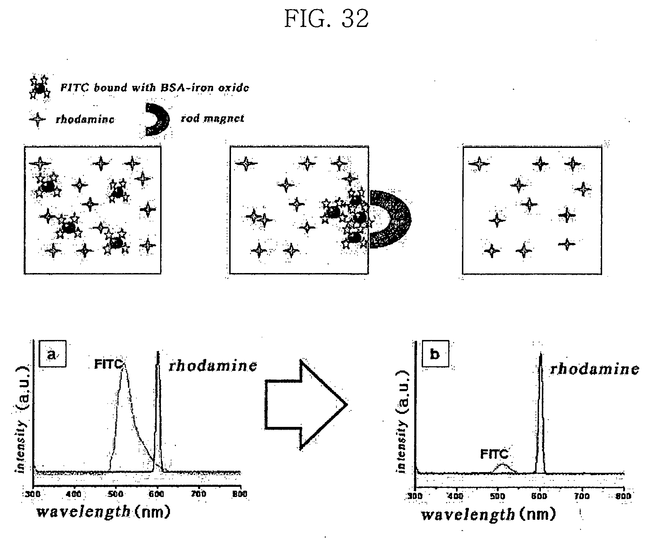

FITC (Fluorescene) bound with the water-soluble iron oxide nanoparticles prepared in Example 49 was mixed with rhodamine not bound with the water-soluble iron oxide nanoparticles, and the fluorescence was measured using a PL spectroscope (FP-6500, Jasco, USA) (FIG. 32A). The iron oxide nanoparticles were collected from the mixture for 30 min using a 0.5 T Co-Sm rod magnet, after which the fluorescence of only the supernatant was measured. As a result, the fluorescence of the FITC was observed to be decreased (FIG. 32B).

### Example 61: Effect of Cancer Treatment using Photocatalytic Activity of Water-Soluble Titanium Dioxide Nanoparticles Coated with Phase Transfer Ligand

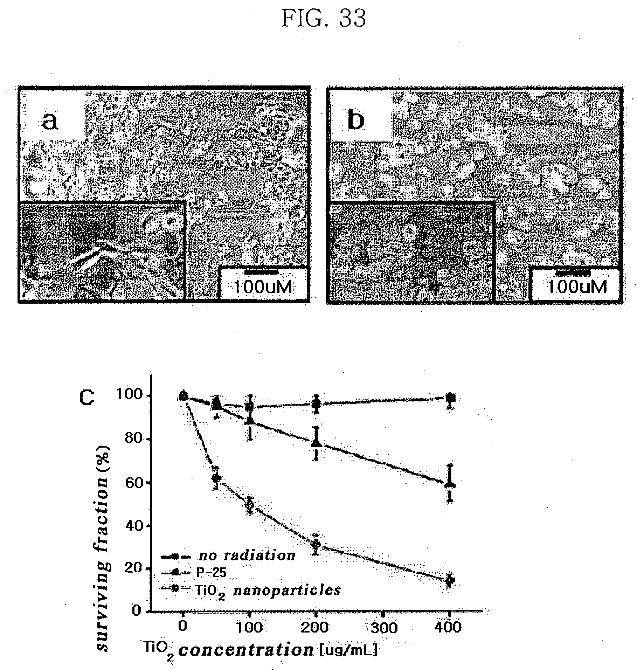

The titanium dioxide nanoparticles synthesized in Example 36 were diluted to concentrations of 50, 100, 200, and 400 mg/ml, injected into skin cancer cells (A-375 in Dulbecco's modified Eagle's medium with 10 % fetal calf serum), and then cultured for 24 hours. After 24 hours, UV light (Selland 2000, 2 kW UV lamp, Germany, 340-440 nm, 20 mW/cm2) was radiated thereon for 30 min, and then the extent of death of the cells was measured through MTT assay. In FIG. 33, a shows the image of the cells after UV light was radiated thereon in the absence of the titanium dioxide nanoparticles, and b in FIG. 33 shows the image of the cells after UV light was radiated thereon in the presence of the titanium dioxide nanoparticles. Further, c in FIG. 33 is a graph showing the degree of treatment of cancer cells using the titanium dioxide nanoparticles coated with phase transfer ligand of the present invention and commercially available P-25. As shown in a and b of FIG. 33, as compared to the skin cancer cells not treated with the titanium dioxide nanoparticles, most of the skin cancer cells treated with the titanium dioxide nanoparticles were observed to be killed. Further, as the concentration of the treated nanoparticles was increased, the effect of cancer treatment was improved, resulting in cancer treatment effects superior to those of commercially available titanium dioxide particles p-25 (c of FIG. 33).

### Industrial Applicability

According to the present invention, water-soluble metal oxide nanoparticles, in which the size and shape thereof are controlled so that they have improved electrical/magnetic properties, can be obtained, and the water-soluble nanoparticles can be used in composite materials, electronic materials, and biomedical diagnosis and treatment, through the introduction of various active components.

## Claims

1. A method of preparing water-soluble metal oxide nanoparticles, comprising the steps of (1) synthesizing water-insoluble metal oxide nanoparticles in an organic solvent; (2) dissolving the water-insoluble metal nanoparticles in a first solvent and dissolving a phase transfer ligand in a second solvent; and (3) mixing the two solutions realized in step (2) to thus exchange the surface of the water-insoluble metal oxide nanoparticles with the phase transfer ligand,
wherein
the phase transfer ligand comprises an adhesive region, an adhesive region-crosslinking region, or an adhesive region-reactive region,
and
the phase transfer ligand is selected from a group consisting of a sugar, a chemical monomer, and a polymer
with the proviso that in case that the metal oxide nanoparticles consist of iron oxide the phase transfer ligand does not comprise an adhesive region.

2. The method of preparing the water-soluble nanoparticles according to 1, wherein the first solvent in step (2) is selected from a group consisting of benzene solvents, hydrocarbon solvents, ether solvents, halohydrocarbons, alcohols, sulfoxide solvents, amide solvents, and ionic liquids.

3. The method of preparing the water-soluble nanoparticles according to 1, wherein the second solvent in step (2) is selected from a group consisting of benzene solvents, hydrocarbon solvents, ether solvents, halohydrocarbons, alcohols, sulfoxide solvents, amide solvents, water, and ionic liquids.

4. The method of preparing the water-soluble nanoparticles according to 1, wherein the metal oxide is MₓO_{y} (Mₓ = one or more metal elements selected from a group consisting of Group 1 and 2 metal elements including Li, Na, Be, Ca, Ge, Ba, Mg, Sr, and Ra, transition metal elements including Sc, Ti, V, Y, Tc, Ru, Rh, Pd, Ag, Cd, Hf, Ta, W, Re, Os, Ir, Pt, and Au, Group 13 elements including Al, Ga and In, Group 14 elements including Ge, Sn, and Pb, Group 15 elements including Bi, and lanthanide and actinide elements including Ce, Pr, Nd, Pm, Er, Tm, Yb, Lu, La, Th, Pa, U, Am, Cm, Bk, Cf, Ex, Fm, Md, No, and Lr; 0<x<20, 0<y<20).

5. The method of preparing the water-soluble nanoparticles according to 1, wherein the adhesive region (L_{I}) of the phase transfer ligand has a functional group selected from a group consisting of -COOH, -NH₂, -SH, -SS-, -CONH₂, -PO₃H,-PO₄H₂, - PO₂(OR₁)(OR₂) (R₁, R₂ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), SO₃H, -SO₄H, -NO₂, -CHO, -COSH, -COX, - COOCO-, -CORCO- (R= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -COOR, -CONH-, -CN, -NROH (R = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁NR₂R₃ (R₁, R₂, R₃ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -CONHNR₁R₂ (R₁, R₂= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2S, 0≤z≤2S), -NR₁R₂R₃X (R₁, R₂, R₃ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), and -OH.

6. The method of preparing the water-soluble nanoparticles according to 1, wherein the crosslinking region (L_{II}) of the phase transfer ligand has a functional group selected from a group consisting of -COOH, -NH₂, -SH, -CONH₂, -PO₃H, -PO₄H₂,-PO₂(OR₁)(OR₂) (R₁, R₂ = CₛHₜNᵤO_{w}P_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), SO₃H, -SO₄H, -NO₂, -CHO, -COSH, -COX, - COOR, -CONH- -CN, -NROH (R = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁NR₂R₃ (R₁, R₂, R₃= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -CONHNR₁R₂ (R₁, R₂= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁R₂R₃X (R₁, R₂, R₃ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -OH, -SCOCH₃, -F, -Cl, -Br, -I, -SCN, -NCO, -OCN, -epoxy, -hydrazone, -alkene, and -alkyne.

7. The method of preparing the water-soluble nanoparticles according to 1, wherein the reactive region (L_{III}) of the phase transfer ligand has a functional group selected from a group consisting of -COOH, -NH₂, -SH, -CONH₂, -PO₃H, -PO₄H₂,-PO₂(OR₁)(OR₂) (R₁, R₂ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), SO₃H, -SO₄H, -NO₂, -CHO, -COSH, -COX, - COOR, -CONH-, -CN, -NROH (R = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁NR₂R₃ (R₁, R₂, R₃= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -CONHNR₁R₂ (R₁, R₂= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁R₂R₃X (R₁, R₂, R₃ = CₛHₜNᵤO_{w}SₓP_{y}Xz, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -OH, -SCOCH₃, -F, -Cl, -Br, -I, -SCN, -NCO, -OCN, -epoxy, hydrazone, -alkene, and -alkyne.

8. The method of preparing the water-soluble nanoparticles according to 1, wherein the sugar is selected from a group consisting of glucose, mannose, fucose, N-acetylglucomin, N-acetylgalactosamin, N-acetylneuramic acid, fructose, levulose, xylose, sorbitol, sucrose, maltose, glycoaldehyde, dihydroxyacetone, erythrose, erythrulose, arabinose, xylose, xylulose, fractose, lactose, maltose, trehalose, melibiose, cellobiose, raffinose, melezitose, maltoriose, stachyose, schrodose, xylan, araban, hesoxan, fructan, galactan, mannan, agaropectin, alginic acid, carrageenan, hemicellulose, dextran, carbodextran, starch, hypromellose, cellulose, amylose, dioxyacetone, glycerinaldehyde, chitin, agarose, dextrin, ribose, riburose, galactose, carboxymethylcellulose, and glycogen.

9. The method of preparing the water-soluble nanoparticles according to 1, wherein the polymer is selected from a group consisting of polyphosphazene, polyacrylic acid, polylactide, polylactide-co-glycolide, polycaprolactone, polycaprolactam, polyanhydride, polymalic acid and derivatives thereof, polyalkylcyanoacrylate, polyhydroxyalkylate, polycarbonate, polyorthoester, polyethyleneglycol, poly-L-lysine, polyglycolide, polyalkylmethacrylate, polyvinylpyrrolidone, and copolymers thereof.

## Patentansprüche

1. Verfahren zum Herstellen wasserlöslicher Metalloxid-Nanopartikel, umfassend die Schritte aus (1) Synthetisieren wasserunlöslicher Metalloxid-Nanopartikel in einem organischen Lösungsmittel; (2) Auflösen der wasserunlöslichen Metall-Nanopartikel in einem ersten Lösungsmittel und Auflösen eines Phasentransfer-Liganden in einem zweiten Lösungsmittel; und (3) Vermischen der beiden in Schritt (2) realisierten Lösungen, um auf diese Weise die Oberfläche der wasserunlöslichen Metalloxid-Nanopartikel mit dem Phasentransfer-Liganden auszutauschen,
wobei
der Phasentransfer-Ligand eine adhäsive Region, eine die adhäsive Region quervernetzende Region oder eine zur adhäsiven Region reaktive Region umfasst,
und
der Phasentransfer-Ligand ausgewählt ist aus einer Gruppe bestehend aus einem Zucker, einem chemischen Monomer und einem Polymer,
mit der Maßgabe, dass im Falle, dass die Metalloxid-Nanopartikel aus Eisenoxid bestehen, der Phasentransfer-Ligand keine adhäsive Region umfasst.

2. Verfahren zum Herstellen der wasserlöslichen Nanopartikel gemäß 1, wobei das erste Lösungsmittel in Schritt (2) aus einer Gruppe ausgewählt ist, die aus Benzol-Lösungsmitteln, Kohlenwasserstoff-Lösungsmitteln, Ether-Lösungsmitteln, halogenierten Kohlenwasserstoffen, Alkoholen, Sulfoxid-Lösungsmitteln, Amid-Lösungsmitteln und ionischen Flüssigkeiten besteht.

3. Verfahren zum Herstellen der wasserlöslichen Nanopartikel gemäß 1, wobei das zweite Lösungsmittel in Schritt (2) aus einer Gruppe ausgewählt ist, die aus Benzol-Lösungsmitteln, Kohlenwasserstoff-Lösungsmitteln, Ether-Lösungsmitteln, halogenierten Kohlenwasserstoffen, Alkoholen, Sulfoxid-Lösungsmitteln, Amid-Lösungsmitteln, Wasser und ionischen Flüssigkeiten besteht.

4. Verfahren zum Herstellen der wasserlöslichen Nanopartikel gemäß 1, wobei das Metalloxid MₓO_{y} ist (Mₓ = ein oder mehrere Metallelemente ausgewählt aus einer Gruppe bestehend aus Metallelementen der Gruppe 1 und 2 einschließlich Li, Na, Be, Ca, Ge, Ba, Mg, Sr, und Ra, Übergangsmetallelementen einschließlich Sc, Ti, V, Y, Tc, Ru, Rh, Pd, Ag, Cd, Hf, Ta, W, Re, Os, Ir, Pt, und Au, Elementen der Gruppe 13 einschließlich Al, Ga und In, Elementen der Gruppe 14 einschließlich Ge, Sn, und Pb, Elementen der Gruppe 15 einschließlich Bi, und Elemente der Lanthanide und Actinide einschließlich Ce, Pr, Nd, Pm, Er, Tm, Yb, Lu, La, Th, Pa, U, Am, Cm, Bk, Cf, Ex, Fm, Md, No, und Lr; 0<x<20, 0<y<20).

5. Verfahren zum Herstellen der wasserlöslichen Nanopartikel gemäß 1, wobei die adhäsive Region (L_{I}) des Phasentransfer-Liganden eine funktionelle Gruppe aufweist, die ausgewählt ist aus einer Gruppe bestehend aus -COOH, -NH₂, -SH, -SS-, -CONH₂, -PO₃H, -PO₄H₂, -PO₂(OR₁)(OR₂) (R₁, R₂ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -SO₃H, -SO₄H, -NO₂ -CHO, -COSH, -COX, -COOCO-, -CORCO- (R= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -COOR, -CONH-, -CN, -NROH (R = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁NR₂R₃ (R₁, R₂, R₃ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -CONHNR₁R₂ (R₁, R₂= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁R₂R₃X (R₁, R₂, R₃ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), und -OH.

6. Verfahren zum Herstellen der wasserlöslichen Nanopartikel gemäß 1, wobei die quervernetzende Region (L_{II}) des Phasentransfer-Liganden eine funktionelle Gruppe aufweist, die ausgewählt ist aus einer Gruppe bestehend aus -COOH, -NH₂, -SH, -CONH₂, -PO₃H, -PO₄H₂, -PO₂(OR₁)(OR₂) (R₁, R₂ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≥y≤2s, 0≤z≤2s), -SO₃H, -SO₄H, -NO₂, -CHO, -COSH, -COX, -COOR, -CONH-, -CN, -NROH (R = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁NR₂R₃ (R₁, R₂, R₃= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -CONHNR₁R₂ (R₁, R₂ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁R₂R₃X (R₁, R₂, R₃ = CₛHₜNᵤO_{w}SₓP_{y}X_{z}, X= -F, -Cl, -Br, -I, 0≤s≤10, 0≤t≤2(s+u)+1, 0≤u<2s, 0≤w≤2s, 0≤x≤2s, 0≤y≤2s, 0≤z≤2s), -OH, -SCOCH₃, -F, -Cl, -Br, -I, -SCN, -NCO, -OCN, -Epoxid, -Hydrazon, -Alken und -Alkin.

7. Verfahren zum Herstellen der wasserlöslichen Nanopartikel gemäß 1, wobei die reaktive Region (L_{III}) des Phasentransfer-Liganden eine funktionelle Gruppe aufweist, die ausgewählt ist aus einer Gruppe bestehend aus -COOH, -NH₂, -SH, -CONH₂, -PO₃H, -PO₄H₂, -PO₂(OR₁)(OR₂) (R₁, R₂ = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -SO₃H, -SO₄H, -NO₂ -CHO, -COSH, -COX, -COOR, -CONH-, -CN, -NROH (R = CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁NR₂R₃ (R₁, R₂, R₃= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -CONHNR₁R₂ (R₁, R₂= CₛHₜNᵤO_{w}SₓP_{y}X_{z}, X= -F, -Cl, -Br, -I, 0≤s≤10, 0≤t≤2(s+u)+1, 0≤u≤2s, 0≤w≤2s, 0≤x≤2s, 0≤y≤2s, 0≤z≤2s), -NR₁R₂R₃X (R₁, R₂, R₃ = CₛHₜNᵤO_{w}SₓP_{y}X_{z}, X= -F, -Cl, -Br, -I, 0≤s≤10, 0≤t<2(s+u)+1, 0≤u<2s, 0≤w≤2s, 0≤x≤2s, 0≤y≤2s, 0≤z≤2s), -OH, -SCOCH₃, -F, -Cl, -Br, -I, -SCN, -NCO, -OCN, -Epoxid, -Hydrazon, -Alken und -Alkin.

8. Verfahren zum Herstellen der wasserlöslichen Nanopartikel gemäß 1, wobei der Zucker ausgewählt ist aus einer Gruppe, die aus Glucose, Mannose, Fucose, N-Acetylglucomin, N-Acetylgalactosamin, N-Acetylneuraminsäure, Fructose, Lävulose, Xylose, Sorbitol, Saccharose, Maltose, Glycoaldehyd, Dihydroxyaceton, Erythrose, Erythrulose, Arabinose, Xylose, Xylulose, Fractose, Lactose, Maltose, Trehalose, Melibiose, Cellobiose, Raffinose, Melezitose, Maltoriose, Stachyose, Schrodose, Xylan, Araban, Hesoxan, Fructan, Galactan, Mannan, Agaropektin, Alginsäure, Carrageenan, Hemicellulose, Dextran, Carbodextran, Stärke, Hypromellose, Cellulose, Amylose, Dioxyaceton, Glycerinaldehyd, Chitin, Agarose, Dextrin, Ribose, Riburose, Galactose, Carboxymethylcellulose und Glykogen besteht.

9. Verfahren zum Herstellen der wasserlöslichen Nanopartikel gemäß 1, wobei das Polymer ausgewählt ist aus einer Gruppe, die aus Polyphosphazen, Polyacrylsäure, Polylactid, Polylactid-co-Glycolid, Polycaprolacton, Polycaprolactam, Polyanhydrid, Polyäpfelsäure und deren Derivate, Polyalkylcyanoacrylat, Polyhydroxyalkylat, Polycarbonat, Polyorthoester, Polyethylenglykol , Poly-L-Lysin, Polyglycolid, Polyalkylmethacrylat, Polyvinylpyrrolidon, und Copolymere davon besteht.

## Revendications

1. Procédé de préparation de nanoparticules d'oxyde métallique hydrosolubles, comprenant les étapes (1) de synthèse de nanoparticules d'oxyde métallique insolubles dans l'eau dans un solvant organique ; (2) de dissolution des nanoparticules métalliques insolubles dans l'eau dans un premier solvant et de dissolution d'un ligand de transfert de phase dans un second solvant ; et (3) de mélange des deux solutions réalisées dans l'étape (2) pour ainsi échanger la surface des nanoparticules d'oxyde métallique insolubles dans l'eau par le ligand de transfert de phase,
dans lequel
le ligand de transfert de phase comprend une région adhésive, une région adhésive/région de réticulation ou une région adhésive/région réactive,
et
le ligand de transfert de phase est choisi dans un groupe constitué par un sucre, un monomère chimique et un polymère,
à condition que dans le cas où les nanoparticules d'oxyde métallique sont constituées d'oxyde de fer, le ligand de transfert de phase ne comprenne pas une région adhésive.

2. Procédé de préparation des nanoparticules hydrosolubles selon la revendication 1, dans lequel le premier solvant dans l'étape (2) est choisi dans un groupe constitué par les solvants benzéniques, les solvants hydrocarbonés, les solvants éthérés, les halohydrocarbures, les alcools, les solvants à base de sulfoxyde, les solvants à base d'amide et les liquides ioniques.

3. Procédé de préparation des nanoparticules hydrosolubles selon la revendication 1, dans lequel le second solvant dans l'étape (2) est choisi dans un groupe constitué par les solvants benzéniques, les solvants hydrocarbonés, les solvants éthérés, les halohydrocarbures, les alcools, les solvants à base de sulfoxyde, les solvants à base d'amide et les liquides ioniques.

4. Procédé de préparation des nanoparticules hydrosolubles selon la revendication 1, dans lequel l'oxyde métallique est MₓO_{y} (Mₓ = un ou plusieurs éléments métalliques choisis dans un groupe constitué par les éléments métalliques des groupes 1 et 2, notamment Li, Na, Be, Ca, Ge, Ba, Mg, Sr et Ra, les éléments métalliques de transition, notamment Sc, Ti, V, Y, Tc, Ru, Rh, Pd, Ag, Cd, Hf, Ta, W, Re, Os, Ir, Pt, et Au, les éléments du groupe 13, notamment Al, Ga et In, les éléments du groupe 14, notamment Ge, Sn et Pb, les éléments du groupe 15, notamment Bi, et les éléments de type lanthanide et actinide, notamment Ce, Pr, Nd, Pm, Er, Tm, Yb, Lu, La, Th, Pa, U, Am, Cm, Bk, Cf, Ex, Fm, Md, No et Lr ; 0 < x <20, 0 < y < 20).

5. Procédé de préparation des nanoparticules hydrosolubles selon la revendication 1, dans lequel la région adhésive (L_{I}) du ligand de transfert de phase contient un groupe fonctionnel choisi dans un groupe constitué par -COOH, -NH₂, -SH, -SS-, -CONH₂, -PO₃H, ⁻PO₄H₂, -PO₂(OR₁) (OR₂) (R₁, R₂ = CₛHₜNᵤO_{w}SₓP_{y}X_{z}, X = -F, -Cl, -Br, -I, 0 ≤ s ≤ 10, 0 ≤ t ≤ 2 (s+u) +1, 0 ≤ u ≤ 2s, 0 ≤ w ≤ 2s, 0 ≤ x ≤ 2s, 0 ≤ y ≤ 2s, 0 ≤ z ≤ 2s), SO₃H, -SO₄H, -NO₂, -CHO, -COSH, -COX, -COOCO-, -CORCO-(R = CₛHₜNᵤO_{w}SₓP_{y}X_{z}, X = -F, -Cl, -Br, -I, 0 ≤ s ≤ 10, 0 ≤ t ≤ 2 (s+u) +1, 0 ≤ u ≤ 2s, 0 ≤ w ≤ 2s, 0 ≤ x ≤ 2s, 0 ≤ y ≤ 2s, 0 z ≤ 2s), -COOR, -CONH-, -CN, -NROH (R = CₛHₜNᵤO_{w}SₓP_{y}X_{z}, X = -F, -Cl, -Br, -I, 0 s ≤ 10, 0 ≤ t ≤ 2 (s+u) +1, 0 ≤ u ≤ 2s, 0 ≤ w ≤ 2s, 0 ≤ x ≤ 2s, 0 ≤ y ≤ 2s, 0 ≤ z ≤ 2s) , -NR₁NR₂R₃ (R₁, R₂, R₃ = CₛHₜNᵤO_{w}SₓP_{y}X_{z}, X = -F, -Cl, -Br, -I, 0 ≤ s ≤ 10, 0 ≤ t ≤ 2 (s+u) +1, 0 ≤ u ≤ 2s, 0 ≤ w ≤ 2s, 0 ≤ x ≤ 2s, 0 ≤ y ≤ 2s, 0 ≤ z ≤ 2s), -CONHNR₁R₂ (R₁, R₂ = CₛHₜNᵤO_{w}SₓP_{y}X_{z}, X = -F, -Cl, -Br, -I, 0 ≤ s ≤ 10, 0 ≤ t ≤ 2 (s+u) +1, 0 ≤ u ≤ 2s, 0 ≤ w ≤ 2s, 0 ≤ x ≤ 2s, 0 ≤ y ≤ 2s, 0 ≤ z ≤ 2s), -NR₁R₂R₃X (R₁, R₂, R₃ = CₛHₜNᵤO_{w}SₓP_{y}X_{z}, X = -F, -Cl, -Br, -I, 0 ≤ s ≤ 10, 0 ≤ t ≤ 2 (s+u) +1, 0 ≤ u ≤ 2s, 0 ≤ w ≤ 2s, 0 ≤ x ≤ 2s, 0 ≤ y ≤ 2s, 0 ≤ z ≤ 2s), et -OH.

6. Procédé de préparation des nanoparticules hydrosolubles selon la revendication 1, dans lequel la région de réticulation (L_{II}) du ligand de transfert de phase contient un groupe fonctionnel choisi dans un groupe constitué par -COOH, -NH₂, -SH, -CONH₂, -PO₃H, -PO₄H₂, -PO₂(OR₁)(OR₂) (R₁, R₂ = CₛHₜNᵤO_{w}SₓP_{y}X_{z}, X = -F, -Cl, -Br, -I, 0 ≤ s ≤ 10, 0 ≤ t ≤ 2 (s+u) +1, 0 ≤ u ≤ 2s, 0 ≤ w ≤ 2s, 0 ≤ x ≤ 2s, 0 ≤ y ≤ 2s, 0 ≤ z ≤ 2s), SO₃H, -SO₄H, -NO₂, -CHO, -COSH, -COX, -COOR, -CONH- -CN, -NROH (R = CₛHₜNᵤO_{w}SₓP_{y}X_{z}, X = -F, -Cl, -Br, -I, 0 ≤ s ≤ 10, 0 ≤ t ≤ 2 (s+u) +1, 0 ≤ u ≤ 2s, 0 ≤ w ≤ 2s, 0 ≤ x ≤ 2s, 0 ≤ y ≤ 2s, 0 ≤ z ≤ 2s), -NR₁NR₂R₃ (R₁, R₂, R₃ = CₛHₜNᵤO_{w}SₓP_{y}X_{z}, X = -F, -Cl, -Br, -I, 0 ≤ s ≤ 10, 0 ≤ t ≤ 2 (s+u) +1, 0 ≤ u ≤ 2s, 0 ≤ w ≤2s, 0 ≤ x ≤ 2s, 0 ≤ y ≤ 2s, 0 ≤ z ≤ 2s), -CONHNR₁R₂ (R₁, + R₂ = CₛHₜNᵤO_{w}SₓP_{y+}X_{z}, X = -F, -Cl, -Br, -I, 0 ≤ s ≤ 10, 0 ≤ t ≤ 2 (s+u) +1, 0 ≤ u ≤ 2s, 0 ≤ w ≤ 2s, 0 ≤ x ≤ 2s, 0 ≤ y ≤ 2s, 0 ≤ z ≤ 2s), -NR₁R₂R₃X (R₁, R₂, R₃ = CₛHₜNᵤO_{w}SₓP_{y}X_{z}, X = -F, -Cl, -Br, -I, 0 ≤ s ≤ 10, 0 ≤ t ≤ 2 (s+u) +1, 0 ≤ u ≤ 2s, 0 ≤ w ≤ 2s, 0 ≤ x ≤ 2s, 0 ≤ y ≤ 2s, 0 ≤ z ≤ 2s), -OH, -SCOCH₃, -F, -Cl, -Br, -I, -SCN, -NCO, -OCN, un époxy, une hydrazone, un alcène et un alcyne.

7. Procédé de préparation de nanoparticules hydrosolubles selon la revendication 1, dans lequel la région réactive (L_{III}) du ligand de transfert de phase contient un groupe fonctionnel choisi dans un groupe constitué par -COOH, -NH₂, -SH, -CONH₂, -PO₃H, -PO₄H₂, -PO₂ (OR₁) (OR₂) (R₁, R₂ = CₛHₜNᵤO_{w}SₓP_{y}X_{z}, X = -F, -Cl, -Br, -I, 0 ≤ s ≤ 10, 0 ≤ t ≤ 2 (s+u) +1, 0 ≤ u ≤ 2s, 0 ≤ w ≤ 2s, 0 ≤ x ≤ 2s, 0 ≤ y ≤ 2s, 0 ≤ z ≤ 2s), SO₃H, -SO₄H, -NO₂, -CHO, -COSH, -COX, -COOR, -CONH-, -CN, -NROH (R = CₛHₜNᵤO_{w}SₓP_{y}X_{z}, X = -F, -Cl, -Br, -I, 0 ≤ s ≤ 10, 0 ≤ t ≤ 2 (s+u) +1, 0 ≤ u ≤ 2s, 0 ≤ w ≤ 2s, 0 ≤ x ≤ 2s, 0 ≤ y ≤ 2s, 0 ≤ z ≤ 2s), -NR₁NR₂R₃ (R₁, R₂, R₃ = CₛHₜNᵤO_{w}SₓP_{y}X_{z}, X = -F, -Cl, -Br, -I, 0 ≤ s ≤ 10, 0 ≤ t ≤ 2(s+u)+1, 0 ≤ u ≤ 2s, 0 ≤ w ≤ 2s, 0 ≤ x ≤ 2s, 0 ≤ y ≤ 2s, 0 ≤ z ≤ 2s), -CONHNR₁R₂ (R₁, R₂ = CₛHₜNᵤO_{w}SₓP_{y}X_{z}, X = -F, -Cl, -Br, -I, 0 ≤ s ≤ 10, 0 ≤ t ≤ 2(s+u)+1, 0 ≤ u ≤ 2s, 0 ≤ w ≤ 2s, 0 ≤ x ≤ 2s, 0 ≤ y ≤ 2s, 0 ≤ z ≤ 2s), -NR₁R₂R₃X (R₁, R₂, R₃ = CₛHₜNᵤO_{w}SₓP_{y}X_{z}, X = -F, -Cl, -Br, -I, 0 ≤ s ≤ 10, 0 ≤ t ≤ 2(s+u)+1, 0 ≤ u ≤ 2s, 0 ≤ w ≤ 2s, 0 ≤ x ≤ 2s, 0 ≤ y ≤ 2s, 0 ≤ z ≤ 2s), -OH, -SCOCH₃, -F, -Cl, -Br, -I, -SCN, -NCO, -OCN, un époxy, une hydrazone, un alcène et un alcyne.

8. Procédé de préparation des nanoparticules hydrosolubles selon la revendication 1, dans lequel le sucre est choisi dans un groupe constitué par le glucose, le mannose, le fucose, la N-acétylglucomine, la N-acétylgalactosamine, l'acide N-acétylneuramique, le fructose, le lévulose, le xylose, le sorbitol, le saccharose, le maltose, le glycoaldéhyde, la dihydroxy-acétone, l'érythrose, l'érythrulose, l'arabinose, le xylose, le xylulose, le fractose, le lactose, le maltose, le tréhalose, le mélibiose, le cellobiose, le raffinose, le mélézitose, le maltoriose, le stachyose, le schrodose, le xylane, l'arabane, l'hesoxane, le fructane, le galactane, le mannane, l'agaropectine, l'acide alginique, le carraghénane, l'hémicellulose, le dextrane, le carbodextrane, l'amidon, l'hypromellose, la cellulose, l'amylose, la dioxyacétone, le glycérinaldéhyde, la chitine, l'agarose, la dextrine, le ribose, le riburose, le galactose, la carboxyméthyl-cellulose et le glycogène.

9. Procédé de préparation des nanoparticules hydrosolubles selon la revendication 1, dans lequel le polymère est choisi dans un groupe constitué par un polyphosphazène, un poly(acide acrylique), un polylactide, un polylactide-co-glycolide, une polycaprolactone, un polycaprolactame, un polyanhydride, un poly(acide malique) et les dérivés de celui-ci, un poly(cyanoacrylate d'alkyle), un poly(hydroxyalkylate), un polycarbonate, un polyorthoester, un polyéthylèneglycol, une poly-L-lysine, un polyglycolide, un poly(méthacrylate d'alkyle), une polyvinylpyrrolidone, et leurs copolymères.
